# EUROPEAN PATENT SPECIFICATION

(11) **EP 4 541 285 B1**
(45) Date of publication and mention of the grant of the patent: **10.12.2025**
(21) Application number: 24158624.7
(22) Date of filing: 20.02.2024
(51) Int. Cl.: A61B 8/00

(54) **ULTRASONIC PROBE**
ULTRASCHALLSONDE
SONDE ULTRASONORE

(30) Priority: 18.10.2023 KR 20230139922
(43) Date of publication of application: 23.04.2025
(73) Proprietor: Samsung Medison Co., Ltd., Hongcheon-gun, Gangwon-do 25108 (KR)
(72) Inventor: LEE, Sungjae, 05377 Seoul (KR); OH, Sangbin, 04971 Seoul (KR)
(74) Representative: Frey, Sven Holger

(56) References cited:
- US-A1- 2008 194 960
- US-A1- 2019 159 758
- US-A1- 2021 275 151

## Description

### BACKGROUND

### 1. Field

The disclosure relates to an ultrasonic probe including an impact reducing structure.

### 2. Description of the Related Art

Recently, in a medical field, various medical imaging devices have been widely used to image and obtain information about biological tissues of a human body for the purpose of early diagnosis of various diseases or surgery. Representative examples of such medical imaging devices may include ultrasonic imaging devices, CT devices, and MRI devices.

An ultrasonic imaging device is a device that emits an ultrasonic signal generated from a transducer of a probe to an object, and non-invasively obtains at least one image of a region inside the object (e.g., soft tissue or blood flow) by receiving information from the signal reflected from the object. An ultrasonic imaging device may be used for medical purposes such as observing the inside of an object, detecting foreign substances, and measuring injury. Such an ultrasonic imaging device is widely used along with other imaging diagnostic devices because the ultrasonic imaging device has higher stability than an imaging device using an X-ray, may display images in real time, and is safe because there is no radiation exposure. In general, an ultrasonic imaging device may include a main body and a probe to transmit an ultrasonic signal to an object to be diagnosed and receiving a signal reflected from the object.

The probe may have a structure in which an ultrasonic signal transmitted from a transducer therein is transmitted to an object by passing through a cap provided to come into contact with the object, and the ultrasonic signal returned by reflection from the object is received to the transducer by passing through the cap again.

Because the cap may has a material such as elastic rubber (RTB), TPE and plastic (synthetic resin, etc.), the cap is vulnerable to external impacts, and thus a user may need to be careful when using the cap.

Because when an impact is applied to the ultrasonic probe, the impact is delivered directly to the transducer accommodated inside the cap, a separate device may be needed to reduce the impact to the transducer.

In particular, because a wire is not connected to a wireless ultrasonic probe, a wireless ultrasonic probe may receive a stronger impact than a wired ultrasonic probe when dropped, and needs to protect key components such as an array module, a battery inside a case, and a beamforming circuit, and thus reducing an impact applied to the wireless ultrasonic probe may be very important.

US 2021/275151 A1 provides an ultrasound probe with thermal and drop impact management.

US 2008/194960 A1 relates to ultrasound imaging systems and more particularly to probes that generate acoustical energy, and receive, process, and transmit information relating to return reflections of the acoustical energy.

US 2019/159758 A1 provides an ultrasonic probe for acquiring an ultrasound image. The ultrasound probe includes a transducer configured to be movable, a cap configured to transmit an ultrasound signal generated by the transducer to outside, and an impact mitigating member disposed along a circumference of the cap to protect the cap from an external impact.

### SUMMARY

It is an aspect of the disclosure to provide an ultrasonic probe with improved durability.

It is an aspect of the disclosure to provide an ultrasonic probe capable of preventing damage to internal components from an external impact.

It is an aspect of the disclosure to provide an ultrasonic probe including an impact reducing member therein.

Additional aspects of the disclosure will be set forth in part in the description which follows and, in part, will be obvious from the description, or may be learned by practice of the disclosure.

The invention is defined by claim 1. In accordance with an aspect of the disclosure, an ultrasonic probe includes a case including a handle part and a head part coupled to the handle part, an acoustic module disposed inside the case to transmit an ultrasonic signal to an object and receive an echo signal reflected from the object, an electronic circuit disposed inside the case to be electrically connected to the acoustic module, and an inner impact reducing member disposed between the acoustic module and the electronic circuit to reduce an impact transmitted to the electronic circuit.

The ultrasonic probe further includes a head impact reducing member disposed between the handle part and the head part.

The inner impact reducing member may further include a flexible circuit board disposed between the acoustic module and the electronic circuit to electrically connect the acoustic module and the electronic circuit.

The ultrasonic probe may further include an energy storage device disposed between the acoustic module and the electronic circuit to supply electric power, wherein the flexible circuit board may be disposed outside the energy storage device to prevent an impact from being transmitted to the energy storage device.

The inner impact reducing member may further include an elastic member disposed between the acoustic module and the electronic circuit.

The ultrasonic probe may further include an energy storage device disposed between the acoustic module and the electronic circuit to supply electric power to the acoustic module or the electronic circuit, wherein the elastic member may be disposed outside the energy storage device to prevent an impact from being transmitted to the energy storage device.

A plurality of the elastic members may be provided, and the plurality of elastic members may be provided to be spaced apart along a longitudinal direction of the energy storage device.

The case may further include a handle part frame disposed inside the handle part to cover the electronic circuit, and a head part frame disposed inside the head part to cover the acoustic module.

The handle part frame and the head part frame may be disposed to be spaced apart from each other.

The ultrasonic probe may further include a case elastic member disposed between the handle part frame and the handle part to reduce the impact transmitted to the electronic circuit.

The ultrasonic probe may further include an energy storage device provided to supply electric power to the acoustic module or the electronic circuit, wherein the energy storage device may be disposed between the handle part frame and the head part frame to be spaced apart from the handle part frame and the head part frame, respectively.

The head part may include a first head part coupled to one side of the handle part and a second head part coupled to the other side of the handle part, and the acoustic module may include a first acoustic module disposed in the first head part and a second acoustic module disposed in the second head part.

The inner impact reducing member may include a first inner impact reducing member disposed between the first acoustic module and the electronic circuit, and a second inner impact reducing member disposed between the second acoustic module and the electronic circuit.

A deformation amount of the inner impact reducing member is provided to be larger than or equal to a deformation amount of the head impact reducing member.

The head impact reducing member is positioned within a section corresponding to the deformation amount of the inner impact reducing member.

In accordance with an aspect of the disclosure, an ultrasonic probe includes a case including a handle part, a first head part coupled to one side of the handle part, and a second head part coupled to the other side of the handle part, an acoustic module provided to transmit an ultrasonic signal to an object and receive an echo signal reflected from the object and including a first acoustic module disposed in the first head part and a second acoustic module disposed in the second head part, an electronic circuit disposed inside the case to be electrically connected to the first acoustic module and the second acoustic module, and an inner impact reducing member provided to reduce an impact transmitted to the electronic circuit and including a first inner impact reducing member disposed between the first acoustic module and the electronic circuit and a second inner impact reducing member disposed between the second acoustic module and the electronic circuit.

The ultrasonic probe may further include a first head impact reducing member disposed between the handle part and the first head part, and a second head impact reducing member disposed between the handle part and the second head part.

The first inner impact reducing member may include a first flexible circuit board disposed between the first acoustic module and the electronic circuit, and the second inner impact reducing member may include a second flexible circuit board disposed between the second acoustic module and the electronic circuit.

The first inner impact reducing member may include a first elastic member disposed between the first acoustic module and the electronic circuit, and the second inner impact reducing member may include a second elastic member disposed between the second acoustic module and the electronic circuit.

The ultrasonic probe may further include an energy storage device provided to supply electric power to the acoustic module and the electronic circuit, wherein the case may include a handle part frame disposed inside the handle part to cover the electronic circuit and the energy storage device.

### BRIEF DESCRIPTION OF THE DRAWINGS

These and/or other aspects of the disclosure will become apparent and more readily appreciated from the following description of the embodiments, taken in conjunction with the accompanying drawings of which:
FIGS. 1A and 1B are block diagrams illustrating components of an ultrasonic imaging system according to an embodiment of the disclosure;
FIGS. 2A, 2B, 2C, and 2D are views illustrating the ultrasonic imaging system according to an embodiment of the disclosure;
FIG. 3 is a perspective view illustrating an ultrasonic probe according to an embodiment of the disclosure;
FIG. 4 is a cross-sectional view illustrating an internal structure of the ultrasonic probe according to an embodiment of the disclosure;
FIG. 5 is a cross-sectional view illustrating a partial structure of the ultrasonic probe including an inner impact reducing member according to an embodiment of the disclosure;
FIG. 6 is an enlarged view of a portion of the ultrasonic probe of FIG. 4;
FIG. 7 is a cross-sectional view illustrating an internal structure of an ultrasonic probe according to an embodiment of the disclosure;
FIG. 8 is an enlarged view of a portion of the ultrasonic probe of FIG. 7, which illustrates a first head side of the ultrasonic probe;
FIG. 9 is a view illustrating the first head side when the ultrasonic probe of FIG. 8 is dropped and impacted;
FIG. 10 is a cross-sectional view of a portion of the ultrasonic probe of FIG. 7, which is viewed from another angle;
FIG. 11 is an enlarged view of another portion of the ultrasonic probe of FIG. 7, which illustrates a second head side of the ultrasonic probe;
FIG. 12 is a view illustrating the second head side when the ultrasonic probe of FIG. 11 is dropped and impacted;
FIG. 13 is a cross-sectional view illustrating an internal structure of an ultrasonic probe according to an embodiment of the disclosure;
FIG. 14 is an enlarged view of a portion of the ultrasonic probe of FIG. 13;
FIG. 15 is a cross-sectional view illustrating an internal structure of the ultrasonic probe according to an embodiment of the disclosure;
FIG. 16 is an enlarged view of a portion of the ultrasonic probe of FIG. 15; and
FIG. 17 is an enlarged view of part A of FIG. 4, which schematically illustrates various shapes capable of being provided as head impact reducing members.

### DETAILED DESCRIPTION

The disclosure explains the principles of the embodiments of the disclosure and discloses the embodiments to clarify the scope of rights of the claims of the disclosure and to enable those skilled in the art in the technical field to which the embodiments of the disclosure belong to practice the embodiments of the disclosure. The embodiments of the disclosure may be implemented in various forms.

Like reference numerals refer to like elements throughout the specification. This specification does not describe all elements of the embodiments, and content that is general in the technical field to which the disclosure belongs or content that is redundant in the embodiments may be omitted. The term 'module' or 'unit' used in the specification may be implemented as one or a combination of two or more of software, hardware, and firmware, and depending on the embodiments, a plurality of 'modules' or 'units' may be implemented as one element, or one 'module' or 'unit' may include a plurality of elements.

The singular form of a noun corresponding to an item may include a single item or a plurality of items, unless the relevant context clearly indicates otherwise.

In the disclosure, each of phrases such as "A or B," "at least one of A and B," "at least one of A or B," "A, B or C," "at least one of A, B and C," and "at least one of A, B, or C" may include any one of the items listed together in the corresponding one of the phrases, or all possible combinations thereof.

The term "and/or" includes any combination of a plurality of related components or any one of a plurality of related components.

The terms such as "first," "second," "primary," and "secondary" may simply be used to distinguish a given component from other corresponding components, and do not limit the corresponding components in any other respect (e.g., importance or order).

The terms "front surface," "rear surface," "upper surface," "lower surface," "side surface," "left side," "right side," "upper portion," "lower portion," and the like used in the disclosure are defined with reference to the drawings, and the shape and position of each component are not limited by these terms.

The terms "comprises," "has," and the like are intended to indicate that there are features, numbers, steps, operations, components, parts, or combinations thereof described in the disclosure, and do not exclude the presence or addition of one or more other features, numbers, steps, operations, components, parts, or combinations thereof.

When any component is referred to as being "connected," "coupled," "supported," or "in contact" with another component, this includes a case in which the components are indirectly connected, coupled, supported, or in contact with each other through a third component as well as directly connected, coupled, supported, or in contact with each other.

When any component is referred to as being located "on" or "over" another component, this includes not only a case in which any component is in contact with another component but also a case in which another component is present between the two components.

Hereinafter, an ultrasonic device according to various embodiments will be described in detail with reference to the accompanying drawings. When described with reference to the attached drawings, similar reference numbers may be assigned to identical or corresponding components and redundant description thereof may be omitted.

In the disclosure, images may include a medical image obtained by a medical imaging device, such as a magnetic resonance imaging (MRI) device, a computed tomography (CT) device, an ultrasonic imaging device, and an x-ray imaging device.

In the disclosure, an "object," which is subject to photography, may include a person, animal, or part thereof. For example, the object may include a part of a human body (organ, etc.) or a phantom.

Throughout the disclosure, an "ultrasonic image" refers to an image of an object generated or processed based on an ultrasonic signal transmitted to and reflected from the object.

Hereinafter, embodiments of the disclosure will be described in detail with reference to the drawings.

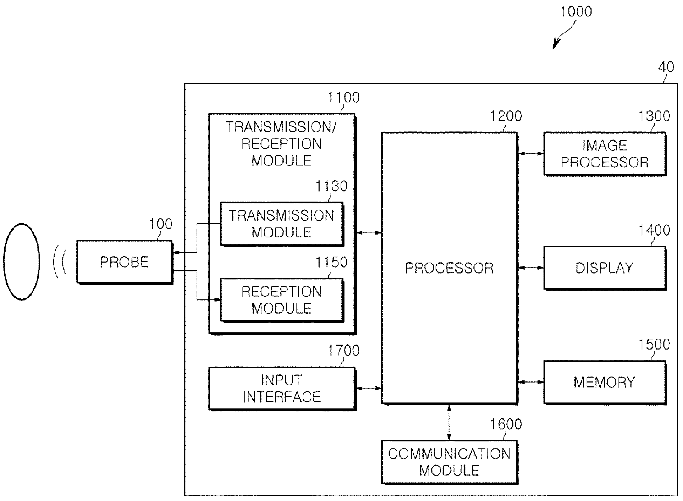

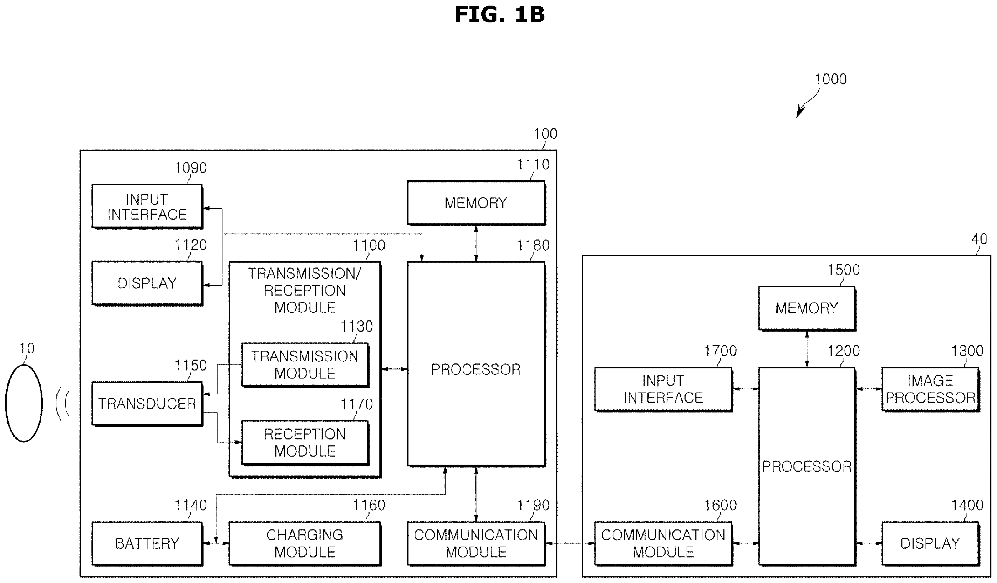

FIGS. 1A and 1B are block diagrams illustrating components of an ultrasonic imaging system according to an embodiment of the disclosure.

Referring to FIGS. 1A and 1B, an ultrasonic imaging system 1000 may include a probe 100 and an ultrasonic imaging device 40.

The ultrasonic imaging device 40 may be implemented not only in a cart type but also in a portable type. A portable ultrasonic imaging device may include, for example, a smart phone, a laptop computer, a personal digital assistant (PDA), or a tablet PC, etc., which includes a probe and an application, but is not limited thereto.

The probe 100 may include a wired probe connected to the ultrasonic imaging device 40 by wire to communicate with the ultrasonic imaging device 40 by wire, a wireless probe wirelessly connected to the ultrasonic imaging device 40 to communicate wirelessly with the ultrasonic imaging device 40, and/or a hybrid probe by wire or wirelessly connected to the ultrasonic imaging device 40 to communicate by wire or wirelessly with the ultrasonic imaging device 40.

According to various embodiments of the disclosure, as illustrated in FIG. 1A, the ultrasonic imaging device 40 may include an ultrasonic transmission/reception module 1100, or as illustrated in FIG. 1B, the probe 100 may include the ultrasonic transmission/reception module 1100. According to various embodiments of the disclosure, both the ultrasonic imaging device 40 and the probe 100 may also include the ultrasonic transmission/reception module 1100.

According to various embodiments of the disclosure, the probe 100 may further include at least one of an image processor 1300, a display 1400, and an input interface 1700, or a combination thereof. In the disclosure, the descriptions of the ultrasonic transmission/reception module 1100, the image processor 1300, the display 1400, or the input interface 1700, which are included in the ultrasonic imaging device 40 may also be applied to the ultrasonic transmission/reception module 1100, the image processor 1300, the display 1400, or the input interface 1700, which are included in the probe 100.

FIG. 1A illustrates a control block diagram illustrating components of the ultrasonic imaging system 1000 in a case in which the probe 100 is a wired probe or a hybrid probe.

The probe 100 may include a plurality of transducers. The plurality of transducers may be arranged in a predetermined arrangement to be implemented as a transducer array. The transducer array may correspond to a one-dimensional (1D) array or a two-dimensional (2D) array. The plurality of transducers may transmit an ultrasonic signal to an object 10 in response to a transmission signal applied from a transmission module 1130. The plurality of transducers may form a reception signal by receiving the ultrasonic signal (echo signal) reflected from the object 10. The probe 100 may be implemented as an integrated type with the ultrasonic imaging device 40, or may be implemented as a separate type connected to the ultrasonic imaging device 40 by wire. The ultrasonic imaging device 40 may be connected to the one or more probes 100 depending on the implementation type.

In the case in which the probe 100 is a wired probe or a hybrid probe, the probe 100 may include a cable and connector capable of being connected to a connector of the ultrasonic imaging device 40.

The probe 100 according to an embodiment of the disclosure may be implemented as a two-dimensional probe. In a case in which the probe 100 is implemented as a two-dimensional probe, the plurality of transducers included in the probe 100 may be arranged in two dimensions to form a two-dimensional transducer array.

For example, the two-dimensional transducer array may have a form in which a plurality of sub-arrays including the plurality of transducers arranged in a first direction is arranged in a second direction different from the first direction.

In addition, in the case in which the probe 20 according to an embodiment of the disclosure is implemented as a two-dimensional probe, the ultrasonic transmission/reception module 1100 may include at least one of an analog beamformer and a digital beamformer. In addition, according to an embodiment of the disclosure, the two-dimensional probe may include at least one of the analog beamformer and the digital beamformer or combination thereof depending on the implementation type.

A processor 1200 controls the transmission module 1130 to form a transmission signal to be applied to each of the transducers 1150 in consideration of positions and focused points of the plurality of transducers included in the probe 100.

The processor 1200 may control a reception module 1150 to generate ultrasonic data by converting reception signals received from the probe 100 to analog to digital and summing up the digitally converted reception signals in consideration of the positions and focused points of the plurality of transducers.

In the case in which the probe 100 is implemented as a two-dimensional probe, the processor 1200 may calculate a time delay value for digital beamforming for each sub-array for each of the plurality of sub-arrays included in the two-dimensional transducer array. The processor 1200 may also calculate a time delay value for analog beamforming for each of the transducers included in one of the plurality of sub-arrays. The processor 1200 may control the analog beamformer and the digital beamformer to form a transmission signal to be applied to each of the plurality of transducers depending on the time delay values for analog beamforming and the time delay values for digital beamforming. The processor 1200 may also control the analog beamformer to sum up the signals received from the plurality of transducers for each sub-array depending on the time delay values for analog beamforming. The processor 1200 may also control the ultrasonic transmission/reception module 1100 to convert the summed signal for each sub-array to analog to digital. The processor 1200 may also control the digital beamformer to generate ultrasonic data by summing up the digitally converted signals depending on the time delay values for digital beamforming.

The image processor 1300 generates or processes an ultrasonic image using the generated ultrasonic data.

The display 1400 may display the generated ultrasonic image and a variety of information processed by the ultrasonic imaging device 40 or the probe 100. The probe 100 or the ultrasonic imaging device 40 may include the one or more displays 1400 depending on the implementation type. The display 1400 may include a touch panel or a touch screen. The display 1400 may also include a flexible display.

The processor 1200 may control the overall operations of the ultrasonic imaging device 40 and control operations of components of the ultrasonic imaging device 40. The processor 1200 may perform or control various operations or functions of the ultrasonic imaging device 40 by executing programs or instructions stored in a memory 1500. The processor 1200 may also control an operation of the ultrasonic imaging device 40 by receiving a control signal from the input interface 1700 or an external device.

The ultrasonic imaging device 40 may include a communication module 1600, and may be connected to and communicate with an external device (e.g., the probe 100, a server, medical device, portable device (a smart phone, tablet PC, wearable device, etc.)) through the communication module 1600.

The communication module 1600 may include one or more components that enable communication with the external device. The communication module 1600 may include, for example, at least one of a short-range communication module, a wired communication module, and a wireless communication module.

The communication module 1600 may receive a control signal or data from the external device. The processor 1200 may control the operation of the ultrasonic imaging device 40 depending on the control signal received through the communication module 1600. In addition, the processor 1200 may transmit the control signal to the external device through the communication module 1600 to control the external device depending on the transmitted control signal. The external device may operate depending on the control signal received from the ultrasonic imaging device 40 or may process the data received from the ultrasonic imaging device 40.

A program or application related to the ultrasonic imaging device 40 may be installed in the external device. The program or application installed in the external device may control the ultrasonic imaging device 40 or operate depending on the control signal or data received from the ultrasonic imaging device 40.

The external device may receive or download the program or application related to the ultrasonic imaging device 40 from the ultrasonic imaging device 40, the probe 100, or a server, and install and execute the program or application in the external device. The ultrasonic imaging device 40, probe 100, or server that provides the program or application may include a recording medium that stores instructions, commands, installation files, executable files, or related data of the program or application. The external device may be sold with the program or application installed.

The memory 1500 may store various data or programs for driving and controlling the ultrasonic imaging device 40, inputted and outputted ultrasonic data, ultrasonic images, and the like.

The input interface 1700 may receive user input for controlling the ultrasonic imaging device 40. For example, the user input may include, but is not limited to, input of manipulating a button, a keypad, a mouse, a trackball, a jog switch, a knob, and the like, input of touching a touch pad or touch screen, voice input, motion input, biometric information input (e.g., iris recognition, fingerprint recognition, etc.), and the like.

FIG. 1B illustrates a control block diagram of the ultrasonic imaging system 1000 in the case in which the probe 100 is a wireless probe or a hybrid probe.

According to various embodiments of the disclosure, the ultrasonic imaging device 40 illustrated in FIG. 1B may be replaced with the ultrasonic imaging device 40 described with reference to FIG. 1A.

According to various embodiments of the disclosure, the probe 100 illustrated in FIG. 1A may be replaced with the probe 100 to be described with reference to FIG. 1B.

The probe 100 may include a display 1120, the transmission module 1130, a battery 1140, the transducer 1150, a charging module 1160, a reception module 1170, an input interface 1090, a processor 1180, and a communication module 1190. Although FIG. 1B illustrates that the probe 100 includes both the transmission module 1130 and the reception module 1170, the probe 100 may include only part of configurations of the transmission module 1130 and the reception module 1170 depending on the implementation type, and the part of the configurations of the transmission module 1130 and the reception module 1170 may be included in the ultrasonic imaging device 40. In addition, the probe 100 may further include the image processor 1300.

The transducer 1150 may include a plurality of transducers. The plurality of transducers may be arranged in a predetermined arrangement to be implemented as a transducer array. The transducer array may correspond to a one-dimensional (1D) array or a two-dimensional (2D) array. The plurality of transducers may transmit an ultrasonic signal to the object 10 in response to the transmission signal applied from the transmission module 1130. The plurality of transducers may also form or generate an electrical reception signal by receiving the ultrasonic signal reflected from the object 10.

The charging module 1160 may charge the battery 1140. The charging module 1160 may receive electric power from the outside. According to an embodiment of the disclosure, the charging module 1160 may receive electric power wirelessly. In addition, according to an embodiment of the disclosure, the charging module 1160 may receive electric power by wire. The charging module 1160 may transfer the received electric power to the battery 1140.

The processor 1180 controls the transmission module 113 to generate or form a transmission signal to be applied to each of the plurality of transducers in consideration of the positions and focused points of the plurality of transducers.

The processor 1180 controls the reception module 1170 to generate ultrasonic data by converting reception signals received from the transducer 1150 to analog to digital and summing up the digitally converted reception signals in consideration of the positions and focused points of the plurality of transducers. According to an embodiment of the disclosure, in a case in which the probe 100 includes the image processor 1300, the probe 100 may generate an ultrasonic image using the generated ultrasonic data.

In the case in which the probe 100 is implemented as a two-dimensional probe, the processor 1180 may calculate a time delay value for digital beamforming for each sub-array for each of the plurality of sub-arrays included in the two-dimensional transducer array. The processor 1180 may also calculate a time delay value for analog beamforming for each of the transducers included in any one of the plurality of sub-arrays. The processor 1180 may control the analog beamformer and the digital beamformer to form a transmission signal to be applied to each of the plurality of transducers depending on the time delay values for analog beamforming and the time delay values for digital beamforming. The processor 1180 may also control the analog beamformer to sum up the signals received from the plurality of transducers for each sub-array depending on the time delay values for analog beamforming. The processor 1180 may also control the ultrasonic transmission/reception module 1100 to convert the summed signal for each sub-array to analog to digital. The processor 1180 may also control the digital beamformer to generate ultrasonic data by summing up the digitally converted signals depending on the time delay values for digital beamforming.

The processor 1180 may control the overall operations of the probe 100 and control operations of components of the probe 100. The processor 1180 may perform or control various operations or functions of the probe 100 by executing programs or instructions stored in a memory 1110. The processor 1180 may also control an operation of the probe 100 by receiving a control signal from the input interface 1090 of the probe 100 or an external device (e.g., the ultrasonic imaging device 40). The processor 1180 may also control an operation of the probe 100 by receiving a control signal from the input interface 1090 or an external device. The input interface 1090 may receive user input for controlling the probe 100. For example, the user input may include, but is not limited to, input of manipulating a button, a keypad, a mouse, a trackball, a jog switch, a knob, and the like, input of touching a touch pad or touch screen, voice input, motion input, biometric information input (e.g., iris recognition, fingerprint recognition, etc.), and the like.

The display 1120 may display an ultrasonic image generated by the probe 100, an ultrasonic image generated by processing ultrasonic data generated in the probe 100, an ultrasonic image received from the ultrasonic imaging device 40, a variety of information processed in the ultrasonic imaging system 1000, and the like. The display 1120 may further display state information of the probe 100. The state information of the probe 100 may include at least one of device information of the probe 100, battery state information of the probe 100, frequency band information of the probe 100, output information of the probe 100, information about whether the probe 100 is abnormal, setting information of the probe 100, and temperature information of the probe 100.

The probe 100 may include the one or more displays 1120 depending on the implementation type. The display 1120 may include a touch panel or a touch screen. The display 1120 may also include a flexible display.

The communication module 1190 may wirelessly transmit the generated ultrasonic data or ultrasonic images to the ultrasonic imaging device 40 through a wireless network. The communication module 1190 may also receive control signals and data from the ultrasonic imaging device 40.

The ultrasonic imaging device 40 may receive the ultrasonic data or ultrasonic images from the probe 100.

In an embodiment of the disclosure, in a case in which the probe 100 includes the image processor 1300 capable of generating an ultrasonic image using the ultrasonic data, the probe 100 may transmit the ultrasonic data or the ultrasonic images generated by the image processor 1300 to the ultrasonic imaging device 40.

In an embodiment of the disclosure, in a case in which the probe 100 does not include the image processor 1300 capable of generating an ultrasonic image using the ultrasonic data, the probe 100 may transmit the ultrasonic data to the ultrasonic imaging device 40. The ultrasonic data may include ultrasonic raw data, and the ultrasonic image may refer to ultrasonic image data.

The ultrasonic imaging device 40 may include the processor 1200, the image processor 1300, the display 1400, the memory 1500, the communication module 1600, and the input interface 1700.

The image processor 1300 generates or processes an ultrasonic image using the ultrasonic data received from the probe 100.

The display 1400 may display an ultrasonic image received from the probe 100, an ultrasonic image generated by processing the ultrasonic data received from the probe 100, a variety of information processed in the ultrasonic imaging system 1000, and the like. The ultrasonic imaging device 40 may include the one or more displays 1400 depending on the implementation type. The display 1400 may include a touch panel or a touch screen. The display 1120 may also include a flexible display.

The processor 1200 may control the overall operations of the ultrasonic imaging device 40 and control the operations of the components of the ultrasonic imaging device 40. The processor 1200 may perform or control various operations or functions of the ultrasonic imaging device 40 by executing the programs or instructions stored in the memory 1500. The processor 1200 may also control the operation of the ultrasonic imaging device 40 by receiving the control signal from the input interface 1700 or an external device.

The ultrasonic imaging device 40 may include the communication module 1600, and may be connected to and communicate with an external device (e.g., the probe 100, a server, medical device, portable device (a smart phone, tablet PC, wearable device, etc.)) through the communication module 1600.

The communication module 1600 may include the one or more components that enable communication with the external device. The communication module 1600 may include, for example, at least one of a short-range communication module, a wired communication module, and a wireless communication module.

The communication module 1600 of the ultrasonic imaging device 40 and the communication module 1190 of the probe 100 may communicate using a network or a short-range wireless communication method. For example, the communication module 1600 of the ultrasonic imaging device 40 and the communication module 1190 of the probe 100 may communicate using any one of wireless LAN, Wi-Fi, Bluetooth, ZigBee, Wi-Fi Direct (WFD), Infrared Data Association (IrDA), Bluetooth Low Energy (BLE), Near Field Communication (NFC), Wireless Broadband Internet (WiBro), World Interoperability for Microwave Access (WiMAX), Shared Wireless Access Protocol (SWAP), Wireless Gigabit Alliance (WiGig), RF communication, a wireless data communication method including 60GHz millimeter wave (mm wave) short-range communication, etc.

To this end, the communication module 1600 of the ultrasonic imaging device 40 and the communication module 1190 of the probe 100 may include at least one of a wireless LAN communication module, a Wi-Fi communication module, a Bluetooth communication module, a ZigBee communication module, a Wi-Fi Direct (WFD) communication module, an Infrared Data Association (IrDA) communication module, a Bluetooth Low Energy (BLE) communication module, a Near Field Communication (NFC) module, a Wireless Broadband Internet (WiBro) communication module, a World Interoperability for Microwave Access (WiMAX) communication module, a Shared Wireless Access Protocol (SWAP) communication module, a Wireless Gigabit Alliance (WiGig) communication module, a RF communication module, and a 60GHz millimeter wave (mm wave) short-range communication module.

In an embodiment of the disclosure, the probe 100 may transmit device information (e.g., ID information) of the probe 100 to the ultrasonic imaging device 40 using a first communication method (e.g., BLE) and may be wirelessly paired with the ultrasonic imaging device 40. The probe 100 may also transmit ultrasonic data and/or ultrasonic images to the paired ultrasonic imaging device 40.

The device information of the probe 100 may include a variety of information related to a serial number, model name, or battery state of the probe 100.

The ultrasonic imaging device 40 may receive the device information (e.g., ID information) of the probe 100 from the probe 100 using the first communication method (e.g., BLE) and may be wirelessly paired with the probe 100. The ultrasonic imaging device 40 may also transmit an activation signal to the paired probe 100 and may receive the ultrasonic data and/or ultrasonic images from the probe 100. In this case, the activation signal may include a signal for controlling the operation of the probe 100.

In an embodiment of the disclosure, the probe 100 may transmit the device information (e.g., ID information) of the probe 100 to the ultrasonic imaging device 40 using the first communication method (e.g., BLE) and may be wirelessly paired with the ultrasonic imaging device 40. The probe 100 may also transmit the ultrasonic data and/or ultrasonic images to the ultrasonic imaging device 40 paired by the first communication method using a second communication method (e.g., 60 GHz millimeter wave and Wi-Fi).

The ultrasonic imaging device 40 may receive the device information (e.g., ID information) of the probe 100 from the probe 100 using the first communication method (e.g., BLE) and may be wirelessly paired with the probe 100. The ultrasonic imaging device 40 may also transmit the activation signal to the paired probe 100 and may receive the ultrasonic data and/or ultrasonic images from the probe 100 using the second communication method (e.g., 60 GHz millimeter wave and Wi-Fi).

According to an embodiment of the disclosure, the first communication method used to pair the probe 100 and the ultrasonic imaging device 40 with each other may have a lower frequency band than a frequency band of the second communication method used by the probe 100 to transmit the ultrasonic data and/or ultrasonic images to the ultrasonic imaging device 40.

The display 1400 of the ultrasonic imaging device 40 may display user interfaces (Uls) indicating the device information of the probe 100. For example, the display 1400 may display the Uls, which indicate identification information of the wireless ultrasonic probe 100, a pairing method indicating the pairing method with the probe 20, a data communication state between the probe 100 and the ultrasonic imaging device 40, a method of performing data communication with the ultrasonic imaging device 40, or the battery state of the probe 100.

In a case in which the probe 100 includes the display 1120, the display 1120 of the probe 100 may display the UI indicating the device information of the probe 100. For example, the display 1120 may display the Uls, which indicate the identification information of the wireless probe 100, the pairing method indicating the pairing method with the probe 100, the data communication state between the probe 100 and the ultrasonic imaging device 40, the method of performing data communication with the ultrasonic imaging device 40, or the battery state of the probe 100.

The communication module 1600 may receive control signals and data from an external device. The processor 1200 may control the operation of the ultrasonic imaging device 40 depending on the control signal received through the communication module 1600.

In addition, the processor 1200 may transmit a control signal to the external device through the communication module 1600, thereby controlling the external device depending on the transmitted control signal. The external device may operate depending on the control signal received from the ultrasonic imaging device 40 or may process the data received from the ultrasonic imaging device 40.

The external device may receive or download the program or application related to the ultrasonic imaging device 40 from the ultrasonic imaging device 40, the probe 100, or a server, and install and execute the program or application in the external device. The ultrasonic imaging device 40, probe 100, or server that provides the program or application may include a recording medium that stores instructions, commands, installation files, executable files, or related data of the program or application. The external device may be sold with the program or application installed.

The memory 1500 may store various data or programs for driving and controlling the ultrasonic imaging device 40, inputted and outputted ultrasonic data, ultrasonic images, and the like.

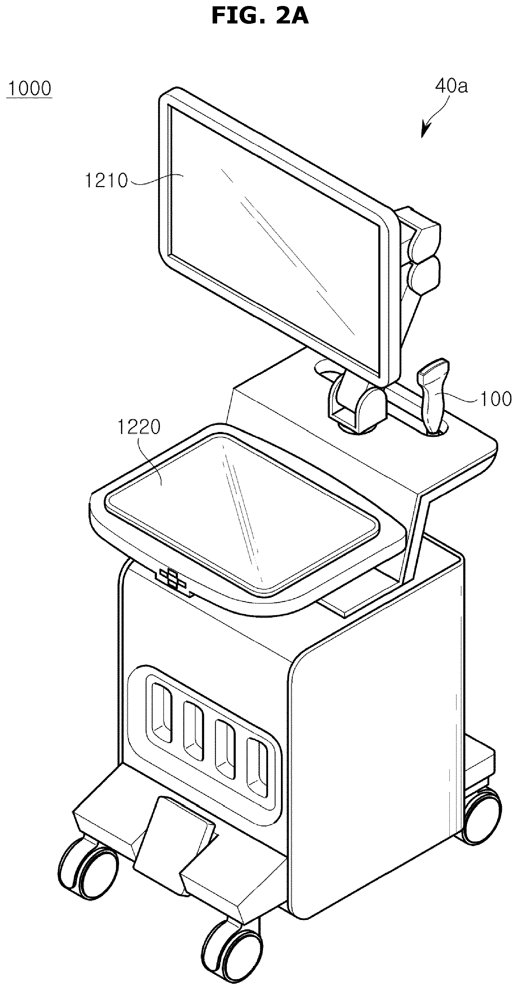

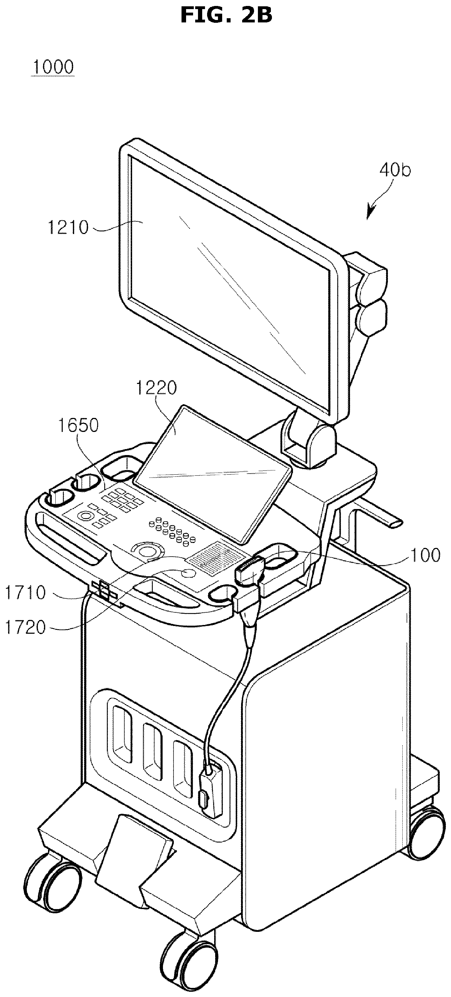

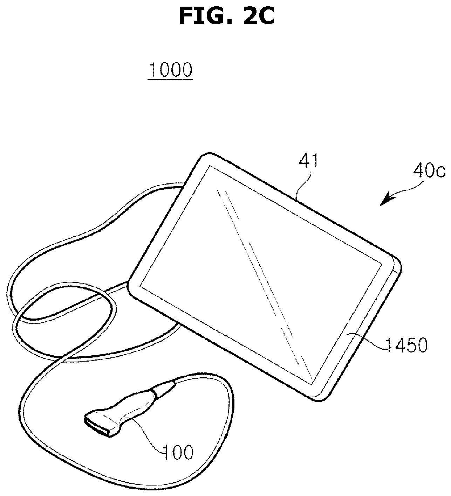

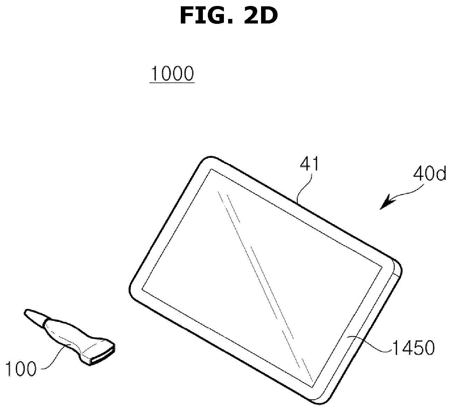

Examples of the ultrasonic imaging system 1000 according to an embodiment of the disclosure will be described later through FIGS. 2A, 2B, 2C, and 2D.

FIGS. 2A, 2B, 2C, and 2D are views illustrating ultrasonic imaging devices 40a, 40b, 40c, and 40d according to an embodiment of the disclosure.

Referring to FIGS. 2A and 2B, the ultrasonic imaging devices 40a and 40b may include a main display 1210 and a sub display 1220. The main display 1210 and sub-display 1220 may correspond to the display 1400 of FIGS. 1A and 1B. At least one of the main display 1210 and the sub display 1220 may be implemented as a touch screen. At least one of the main display 1210 and the sub display 1220 may display ultrasonic images or a variety of information processed by the ultrasonic imaging devices 40a and 40b. In addition, at least one of the main display 1210 and the sub display 1220 may be implemented as a touch screen and provide a graphic user interface (GUI), so that data for controlling the ultrasonic imaging devices 40a and 40b may be inputted thereto from a user. For example, the main display 1210 may display an ultrasonic image, and the sub display 1220 may display a control panel for controlling the display of the ultrasonic image in the form of a GUI. Data for controlling the display of images may be inputted to the sub display 1220 through the control panel displayed in the form of a GUI. For example, a time gain compensation (TGC) button, a lateral gain compensation (LGC) button, a Freeze button, a trackball, a jog switch, a knob, and the like may be provided as GUIs on the sub display 1220.

The ultrasonic imaging devices 40a and 40b may control the display of ultrasonic images displayed on the main display 1210 using the inputted control data. The ultrasonic imaging devices 40a and 40b may also be connected to the probe 100 by wire or wirelessly to transmit and receive ultrasonic signals to and from the object 10.

Referring to FIG. 2B, the ultrasonic imaging device 40b may further include a control panel 1650 in addition to the main display 1210 and the sub display 1220. The control panel 1650 may include a button, a trackball, a jog switch, a knob, and the like, and the data for controlling the ultrasonic imaging device 40b may be inputted to the control panel 1650 from the user. For example, the control panel 1650 may include a time gain compensation (TGC) button 1710, a Freeze button 1720, and the like. The TGC button 1710 is a button for setting a TGC value for each depth of the ultrasonic images. When the input of the Freeze button 1720 is sensed while scanning an ultrasonic image, the ultrasonic imaging device 40b may keep a state in which a frame image at a corresponding point in time is displayed, capture the frame image at the corresponding point in time, or store the frame image at the corresponding point in time.

A button, a trackball, a jog switch, a knob, and the like included in the control panel 1650 may be provided on the main display 1210 or the sub display 1220 as the GUIs. The ultrasonic imaging devices 40a and 40b may be connected to the probe 100 to transmit and receive ultrasonic signals to and from the object 10.

The ultrasonic imaging devices 40a and 40b may include various types of input/output interfaces such as speakers, LEDs, and vibration devices. For example, the ultrasonic imaging devices 40a and 40b may output a variety of information in the form of graphics, sound, or vibration through input/output interfaces. The ultrasonic imaging devices 40a and 40b may also output various notifications or data through the input/output interfaces.

Referring to FIGS. 2C and 2D, the ultrasonic imaging devices 40c and 40d may also be implemented in a portable form. The portable ultrasonic imaging devices 40c and 40d may include, for example, smart phones, laptop computers, or PDAs, tablet PCs, which include a probe and an application, but are not limited thereto.

The ultrasonic imaging device 40c may include a main body 41. Referring to FIG. 2C, the probe 100 may be connected to one side of the main body 41 by wire. To this end, the main body 41 may include a connection terminal to and from which a cable connected to the probe 100 is attachable and detachable. The probe 100 may include a cable including a connection terminal capable of being connected to the main body 41.

Referring to FIG. 2D, the probe 100 may be wirelessly connected to the ultrasonic imaging device 40d. The main body 41 may include an input/output interface (e.g., a touch screen). Ultrasonic images, a variety of information processed by the ultrasonic imaging device, GUIs, and the like may be displayed on the input/output interface 155.

The ultrasonic imaging device 40d and the probe 100 may establish communication or be paired using short-range wireless communication. For example, the ultrasonic imaging device 40d and the probe 100 may communicate using Bluetooth, BLE, Wi-Fi, or Wi-Fi Direct.

The ultrasonic imaging devices 40c and 40d may execute a program or application related to the probe 100 to control the probe 100 and output information related to the probe 100. The ultrasonic imaging devices 40c and 40d may perform operations related to the probe 100 while communicating with a predetermined server. The probe 100 may be registered with the ultrasonic imaging devices 40c and 40d or may be registered with the predetermined server. The ultrasonic imaging devices 40c and 40d may communicate with the registered probe 100 and perform the operations related to the probe 100.

The ultrasonic imaging devices 40c and 40d may include various types of input/output interfaces such as speakers, LEDs, and vibration devices. For example, the ultrasonic imaging devices 40c and 40d may output a variety of information in the form of graphics, sound, or vibration through input/output interfaces. The ultrasonic imaging devices 40c and 40d may also output various notifications or data through the input/output interfaces.

According to an embodiment of the disclosure, the ultrasonic imaging device 40a, 40b, 40c, or 40d may process an ultrasonic image or obtain additional information from the ultrasonic image, using an artificial intelligence (AI) model. According to an embodiment of the disclosure, the ultrasonic imaging device 40a, 40b, 40c, or 40d may generate an ultrasonic image or perform processing such as correction, image quality improvement, encoding, and decoding on the ultrasonic image, using the Al model. In addition, according to an embodiment of the disclosure, the ultrasonic imaging device 40a, 40b, 40c, or 40d may perform processing such as defining a baseline, obtaining anatomical information, obtaining lesion information, extracting a surface, defining a boundary, measuring a length, measuring an area, measuring a volume, or generating an annotation from an ultrasonic image, using the Al model.

The Al model may be provided on the ultrasonic imaging device 40a, 40b, 40c, or 40d, or may be provided on a server.

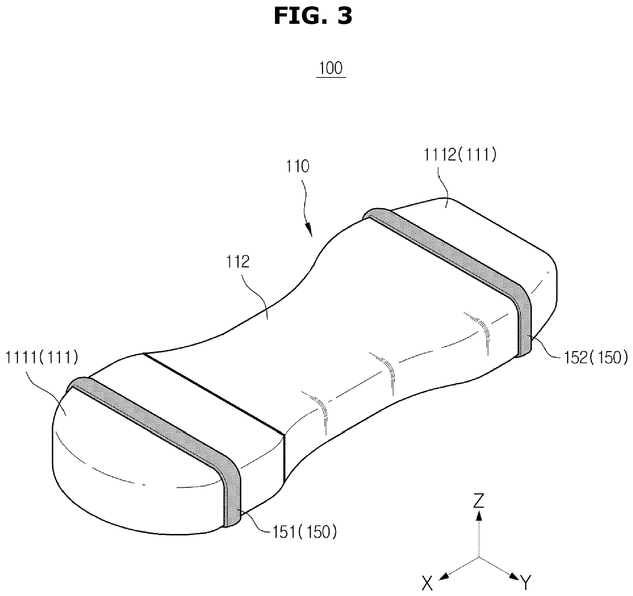

The Al model may be implemented using various artificial neural network models or deep neural network models. In addition, The AI model may be learned and created using various machine learning algorithms or deep learning algorithms. The Al model may be implemented using, for example, a convolutional neural network (CNN), a recurrent neural network (RNN), a generative adversarial network (GAN), or a long short-term memory (LSTM). FIG. 3 is a perspective view illustrating an ultrasonic probe according to an embodiment of the disclosure. A direction or the like may be explained with reference to an X-axis, Y-axis, and Z-axis of FIG. 3.

Referring to FIG. 3, the ultrasonic probe 100 may include a case 110. The case 110 may include a handle part 112 provided to allow the user to hold the ultrasonic probe 100. The handle part 112 may accommodate internal components of the ultrasonic probe 100.

The case 110 may include a head part 111 provided at one end of the handle part 112. The handle part 112 may be coupled to the head part 111. The head part 111 may be disposed on one or opposite sides of the handle part 112.

The head part 111 may include a first head part 1111 provided on one side of the handle part 112 and a second head part 1112 provided on the other side of the handle part 112.

The ultrasonic probe 100 may include a head impact reducing member 150 disposed between the handle part 112 and the head part 111. The head impact reducing member 150 may include a material having elasticity to reduce an impact applied from the outside. For example, the head impact reducing member 150 may include RVT silicone, urethane, rubber, TPE, TPU, etc.

Specifically, the head impact reducing member 150 may include a first head impact reducing member 151 disposed between the first head part 1111 and the handle part 112, and a second head impact reducing member 152 disposed between the second head part 1112 and the handle part 112. That is, the first head impact reducing member 151 may be disposed on one side of the handle part 112. Also, the second head impact reducing member 152 may be disposed on the other side of the handle part 112.

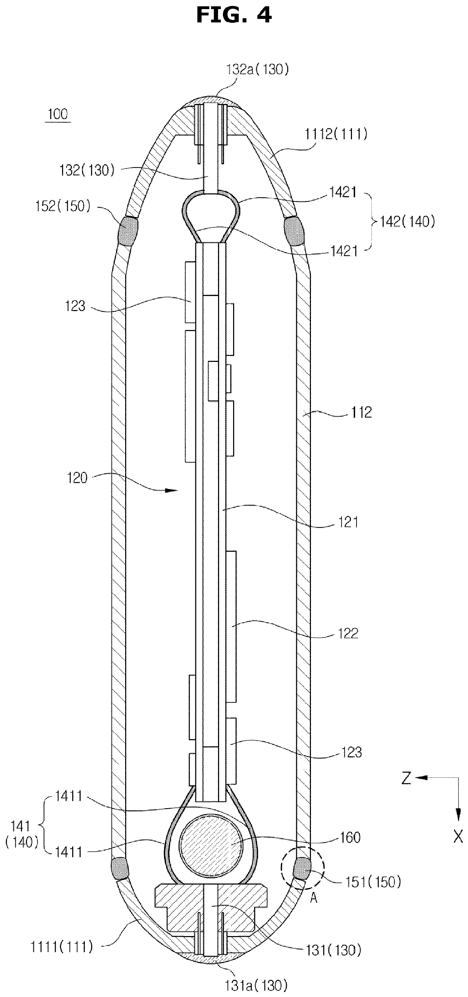

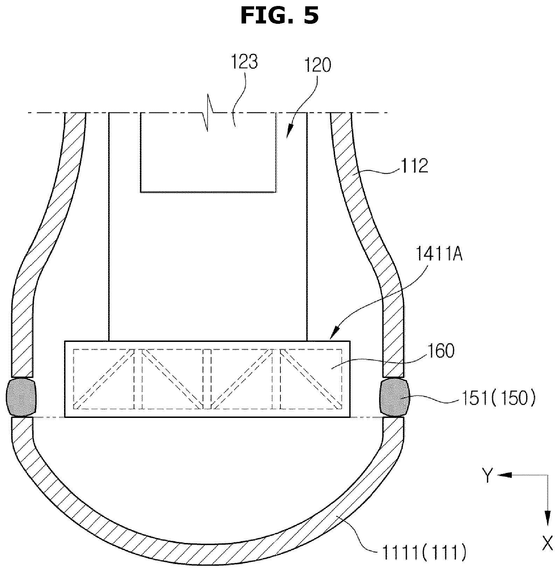

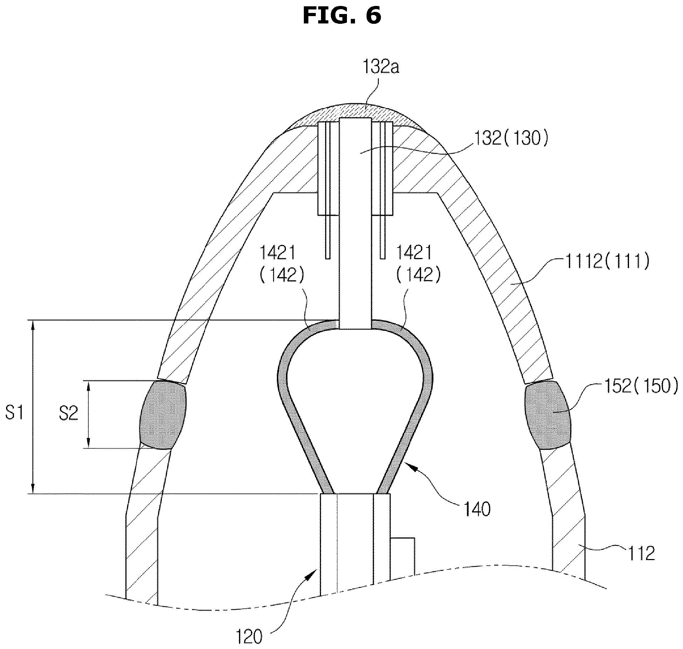

FIG. 4 is a cross-sectional view illustrating an internal structure of the ultrasonic probe according to an embodiment of the disclosure. FIG. 5 is a cross-sectional view illustrating a partial structure of the ultrasonic probe including an inner impact reducing member according to an embodiment of the disclosure. FIG. 6 is an enlarged view of a portion of the ultrasonic probe of FIG. 4. FIG. 4 illustrates a cross section cut along an XZ plane, and FIG. 5 illustrates a cross section cut along an XY plane.

Referring to FIGS. 4 to 6, the ultrasonic probe 100 may include an electronic circuit 120 and an acoustic module 130 disposed inside the case 110. The electronic circuit 120 may be electrically connected to the sound module 130.

The acoustic module 130 may transmit an ultrasonic signal to an object and receive an echo signal reflected from the object. The sound module 130 may be disposed on the head part 111. Specifically, the sound module 130 may be disposed inside the head part 111.

The acoustic module 130 may include a first acoustic module 131 disposed on the first head part 1111 and a second acoustic module 132 disposed on the second head part 1112. The first acoustic module 131 may be arranged in a convex shape, and the second acoustic module 132 may be arranged in a linear shape. Correspondingly, the first head part 1111 may have a convex shape, and the second head part 1112 may have a linear shape.

The acoustic module 130 may include acoustic lenses 131a and 132a provided to transmit an ultrasonic signal generated from the acoustic module 130 to the outside. The acoustic lenses 131a and 132a may focus an ultrasonic signal.

The acoustic lenses 131a and 132a may be made of a material such as silicone and rubber having an acoustic impedance value similar to an acoustic impedance of the object. The acoustic lenses 131a and 132a may be provided as convex types formed to have a central portion having a convex curved surface, or may be provided as linear types having a flat surface.

The acoustic lenses 131a and 132a may include the first acoustic lens 131a disposed on the first acoustic module 131 side and the second acoustic lens 132a disposed on the second acoustic module 132 side, respectively.

The arrangement of an array constituting the acoustic module 130 and the shape of the head part 111 are not limited thereto. For example, the first acoustic module 131 may be arranged in a linear shape, and the second acoustic module 132 may be arranged in a convex shape. Correspondingly, the first acoustic lens 131a may be provided in a linear shape, and the second acoustic lens 132a may be provided in a convex shape.

The electronic circuit 120 may include a main board 121, a power board 122 provided to supply electric power, and a beamformer 123 for focusing echo ultrasonic signals or ultrasonic signals. The electronic circuit 120 may include other components for the ultrasonic probe 100 to operate normally.

The electronic circuit 120 may be disposed inside the handle part 112. That is, the main board 121, power board 122, beamformer 123, and the like may be disposed inside the handle part 112. However, this is only an example, and the arrangement of components inside the case 110 may be changed depending on design specifications.

The electronic circuit 120 may be coupled to the handle part 112 through a frame or the like. The electronic circuit 120 may be fixed to the handle part 112 to move integrally with the handle part 112. Therefore, when the ultrasonic probe 100 is dropped and receives an impact from the outside, the impact may also be transmitted to the electronic circuit 120 inside the ultrasonic probe 100.

The acoustic module 130 may be coupled to the head part 111. That is, the acoustic module 130 may be fixed to the head part 111 to move integrally with the head part 111. Specifically, the first acoustic module 131 may be coupled to the first head part 1111 to move as one body, and the second acoustic module 132 may be coupled to the second head part 1112 to move as one body. Therefore, when an impact is applied to the head part 111, the impact may be transmitted to the acoustic module 130.

The ultrasonic probe 100 may include an energy storage device 160 provided to supply electric power. The energy storage device 160 may be disposed between the acoustic module 130 and the electronic circuit 120. However, the disposition of the energy storage device 160 is not limited thereto.

The energy storage device 160 may be composed of at least one of lithium-ion (Li-ion), nickel metal hydroxide (Ni-MH), lead oxide (PbOx), and sodium-sulfur (Na-S). However, the energy storage device 160 is not limited to the above-described example, and may be made of a rechargeable substance and/or material such as lithium metal oxide, an organic electrode material, and a transition metal.

When an external impact is applied to the ultrasonic probe 100, the impact may also be transmitted to the energy storage device 160 disposed inside the ultrasonic probe 100. When the energy storage device 160 receives a strong impact, damage or fire may occur. Or, problems may occur in ultrasonic output or control functions.

In order to prevent an impact from being transmitted to the electronic circuit 120 and/or the energy storage device 160, the ultrasonic probe 100 may include an inner impact reducing member 140 provided to reduce the impact transmitted to the electronic circuit 120.

The inner impact reducing member 140 may be disposed inside the case 110 to reduce an impact applied to the ultrasonic probe 100. The inner impact reducing member 140 may be disposed between the acoustic module 130 and the electronic circuit 120.

The energy storage device 160 may be located inside the inner impact reducing member 140. That is, while the inner impact reducing member 140 absorbs an impact, the impact may be prevented from being transmitted to the energy storage device 160.

The inner impact reducing member 140 may include a first inner impact reducing member 141 disposed between the first acoustic module 131 and the electronic circuit 120, and a second inner impact reducing member 142 disposed between the second acoustic module 132 and the electronic circuit 120.

The first and second inner impact reducing members 141 and 142 may include flexible circuit boards 1411 and 1421 provided to electrically connect the acoustic modules 131 and 132 and the electronic circuit 120, respectively. However, the first and second inner impact reducing members 141 and 142 are not limited thereto, and may be provided to include wires electrically connecting the first and second acoustic modules 131 and 132 and the electronic circuit 120, and thin plastic plates with elasticity, respectively.

The flexible printed circuit boards 1411 and 1421 may include the first flexible printed circuit board 1411 provided to electrically connect the first acoustic module 131 and the electronic circuit 120, and the second flexible printed circuit board 1421 provided to electrically connect the second acoustic module 132 and the electronic circuit 120.

The energy storage device 160 may be disposed between the first acoustic module 131 and the electronic circuit 120. That is, the first flexible circuit board 1411 may be disposed to surround the energy storage device 160.

The flexible circuit boards 1411 and 1421 may have elasticity. The flexible circuit boards 1411 and 1421 may be formed of a flexible material. That is, when an impact is applied, the flexible circuit boards 1411 and 1421 may absorb the impact while being elastically deformed.

The first flexible circuit board 1411 and the second flexible circuit board 1421 may each be provided as a pair. The first flexible circuit board 1411 and the second flexible circuit board 1421 may also be provided to be symmetrical with respect to a center of the ultrasonic probe 100.

Referring to FIG. 5, the first inner impact reducing member 141 may include an elastic case 1411A disposed outside the energy storage device 160.

The elastic case 1411A may be disposed to surround the energy storage device 160. That is, the energy storage device 160 may be accommodated inside the elastic case 1411A.

The elastic case 1411A may include an elastic material. The elastic case 1411A may be elastically deformed when an impact is applied. That is, the elastic case 1411A may absorb the impact.

The elastic case 1411A may be formed in a truss structure to have structural stability. However, the elastic case 1411A is not limited thereto, and may be provided in any shape as long as it may accommodate the energy storage device 160. For example, the elastic case 1411A may be provided in a grid shape.

A case in which the ultrasonic probe 100 is dropped and the second head part 1112 is impacted will be described below as an example with reference to FIG. 6.

The impact applied to the second head part 1112 may be absorbed by the second head impact reducing member 152 and the second flexible circuit board 1421 of the inner impact reducing member 142.

A deformation amount of the second flexible circuit board 1421 may be provided to be larger than or equal to a deformation amount of the second head impact reducing member 152. The deformation amount of the second flexible circuit board 1421 may be provided to be larger than or equal to the deformation amount of the second head impact reducing member 152 in order to protect the internal components of the ultrasonic probe 100 from an external impact.

In this case, a deformation section S1 of the second flexible circuit board 1421 may be provided to be longer than or equal to a deformation section S2 of the second head impact reducing member 152. In addition, the deformation section S2 of the second head impact reducing member 152 may be provided to be positioned within the deformation section S1 of the second flexible printed circuit board 1421.

In other words, the deformation section S1 of the inner impact reducing member 140 may be provided to be longer than or equal to the deformation section S2 of the head impact reducing member 150.

A thickness of the inner impact reducing member 140 may be provided to be thicker than or equal to a thickness of the head impact reducing member 150. In addition, the thickness of the head impact reducing member 150 may be positioned within a section corresponding to the thickness of the inner impact reducing member 140.

Herein, the thickness of the inner impact reducing member 140 may be a length occupied by the inner impact reducing member 140 along a longitudinal direction of the ultrasonic probe 100. The thickness of the head impact reducing member 150 may be a length occupied by the head impact reducing member 150 along the longitudinal direction of the ultrasonic probe 100.

As such, when the deformation section S1 of the inner impact reducing member 140 is longer than or equal to the deformation section S2 of the head impact reducing member 150 or the deformation section S2 of the head impact reducing member 150 is positioned within the deformation section S1 of the inner impact reducing member 140, the deformation amount of the inner impact reducing member 140 may be larger than or equal to the deformation amount of the head impact reducing member 150. Therefore, an impact transmitted to the internal components such as the electronic circuit 120 may be reduced more efficiently.

Hereinafter, an embodiment different from the ultrasonic probe 100 illustrated in FIGS. 1 to 4 will be described. The same reference numerals may be assigned to components that are the same as the components of the ultrasonic probe illustrated in FIGS. 1 to 4 and descriptions thereof may be omitted, and different components will be described in detail.

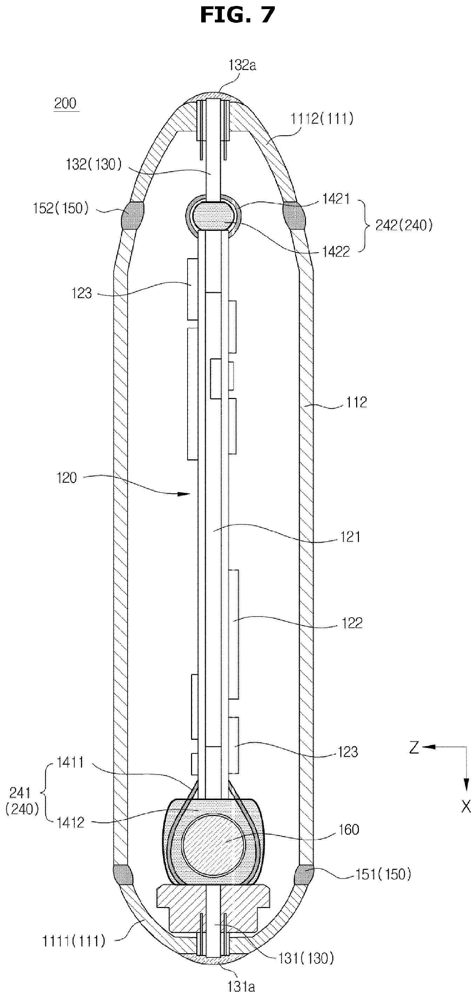

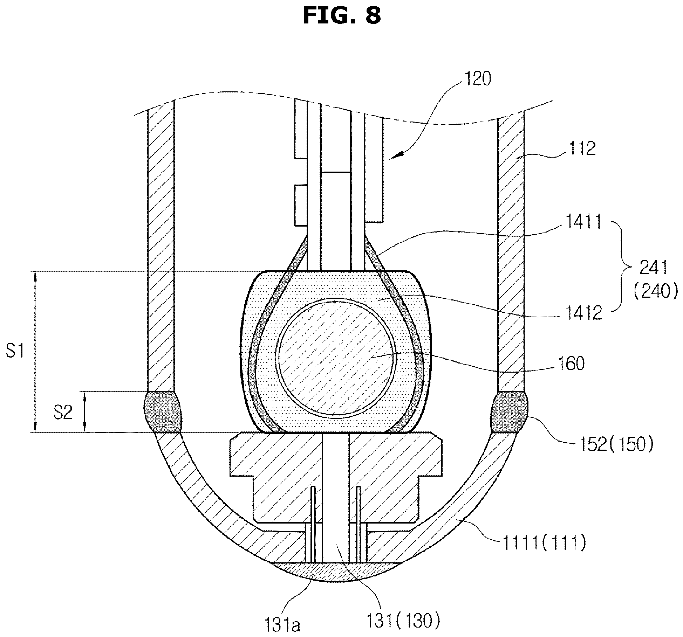

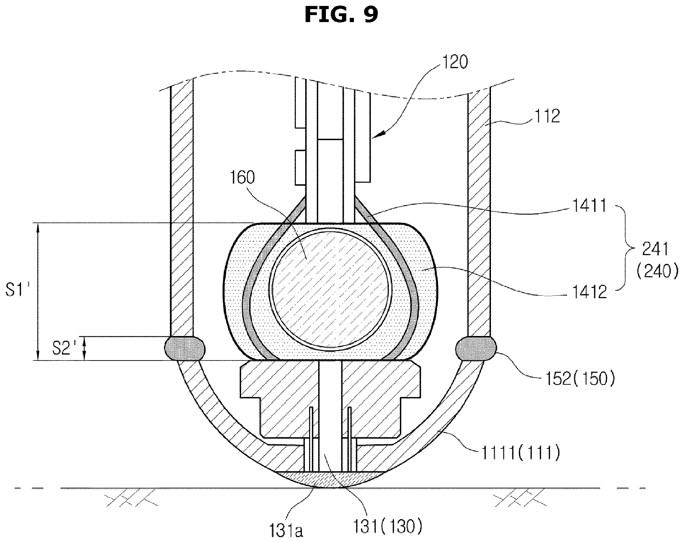

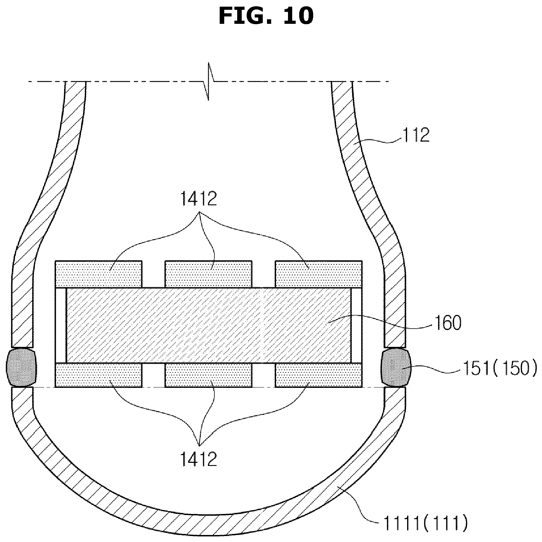

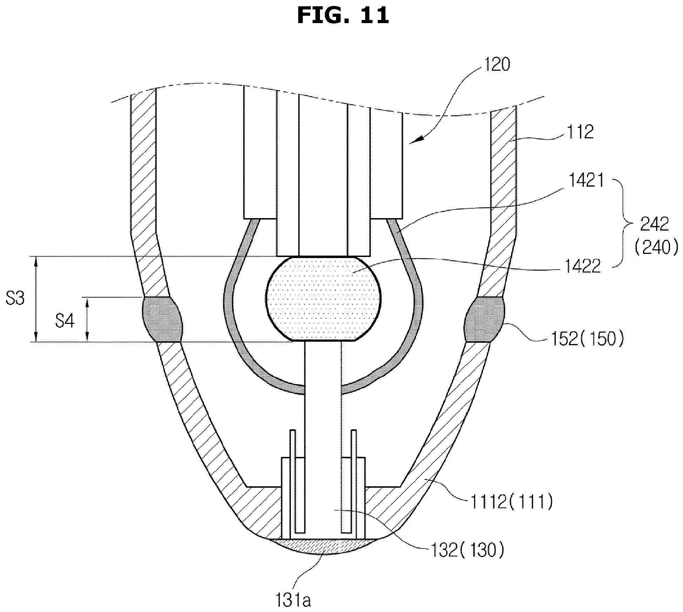

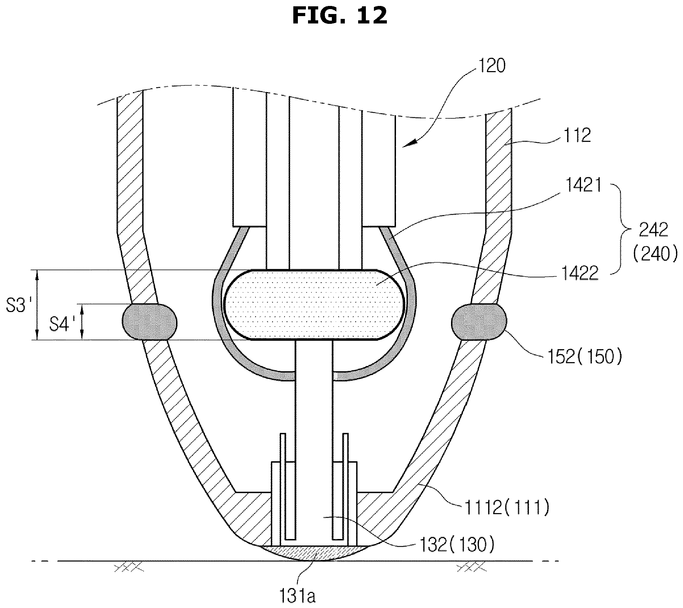

FIG. 7 is a cross-sectional view illustrating an internal structure of an ultrasonic probe according to an embodiment of the disclosure. FIG. 8 is an enlarged view of a portion of the ultrasonic probe of FIG. 7, which illustrates a first head side of the ultrasonic probe. FIG. 9 is a view illustrating the first head side when the ultrasonic probe of FIG. 8 is dropped and impacted. FIG. 10 is a cross-sectional view of a portion of the ultrasonic probe of FIG. 7, which is viewed from another angle. FIG. 11 is an enlarged view of another portion of the ultrasonic probe of FIG. 7, which illustrates a second head side of the ultrasonic probe. FIG. 12 is a view illustrating the second head side when the ultrasonic probe of FIG. 11 is dropped and impacted.

Referring to FIGS. 7 to 12, the ultrasonic probe 200 may include an inner impact reducing member 240 disposed between the acoustic module 130 and the electronic circuit 120.

The inner impact reducing member 240 may include the flexible circuit boards 1411 and 1421 provided to electrically connect the acoustic module 130 and the electronic circuit 120, and elastic members 1412 and 1422 disposed between the acoustic module 130 and the electronic circuit 120.

The elastic members 1412 and 1422 may include a material having elasticity to reduce an impact applied from the outside. The elastic members 1412 and 1422 may include the same material as the head impact reducing members 151 and 152. However, the elastic members 1412 and 1422 are not limited thereto and may be formed of different materials. For example, the elastic members 1412 and 1422 may include elastic bodies made of a metal material such as RTV silicone, urethane, rubber, TPE, TPU, a coil spring, and a leaf spring. The elastic members 1412 and 1422 may be formed of different materials or may be formed of the same material.

Specifically, the inner impact reducing member 240 may include a first inner impact reducing member 241 disposed between the first acoustic module 131 and the electronic circuit 120, and a second inner impact reducing member 242 disposed between the second acoustic module 132 and the electronic circuit 120.

The first inner impact reducing member 241 may include a first flexible circuit board 1411 provided to electrically connect the first acoustic module 131 and the electronic circuit 120.

The first inner impact reducing member 241 may prevent an impact from being transmitted to the energy storage device 160 by being disposed outside the energy storage device 160. The first inner impact reducing member 241 may include the first elastic member 1412 provided to prevent an impact from being transmitted to the energy storage device 160.

Referring to FIG. 10, the energy storage device 160 may be provided as a cylindrical battery with a predetermined length. However, the energy storage device 160 is not limited thereto and may be provided in various shapes such as a button type and square shape. In the disclosure, a case in which the energy storage device 160 has the predetermined length, such as a cylindrical battery, will be described.

As illustrated in FIG. 9, a plurality of the first elastic members 1412 may be provided. The plurality of first elastic members 1412 may be arranged along a longitudinal direction of the energy storage device 160. However, in a case in which the energy storage device 160 does not have a sufficient length, the single first elastic member 1412 may be provided.

The first elastic member 1412 may be disposed outside the energy storage device 160. The first elastic member 1412 may be provided to surround at least a portion of the energy storage device 160. It is sufficient for the first elastic member 1412 and the first flexible circuit board 1411 to be arranged such that the first elastic member 1412 and the first flexible circuit board 1411 do not interfere with each other. For example, the first flexible circuit board 1411 may be positioned between the first elastic members 1412.

Referring to FIGS. 8 and 9, the first inner impact reducing member 241 may double protect the energy storage device 160 through the first flexible circuit board 1411 and the first elastic member 1412. That is, the first inner impact reducing member 241 may be configured to reduce the impacts of the first flexible circuit board 1411 and the first elastic member 1412, respectively.

A deformation amount of the first inner impact reducing member 241 may be provided to be larger than or equal to a deformation amount of the first head impact reducing member 151. The first inner impact reducing member 241 may be provided to have a larger deformation amount in order to protect the internal components of the ultrasonic probe 200 from an external impact.

In this case as well, the deformation section S1 of the first inner impact reducing member 241 may be provided to be longer than or equal to the deformation section S2 of the first head impact reducing member 151. In addition, a thickness of the first inner impact reducing member 241 may be provided to be thicker than a thickness of the first head impact reducing member 151.

The first head impact reducing member 151 may be positioned within the deformation section S1 of the first inner impact reducing member 241. That is, the first head impact reducing member 151 may be positioned within a section corresponding to the thickness of the first inner impact reducing member 241.

That is, the first inner impact reducing member 241 inside the case 110 may be configured to occur a deformation amount equal to the deformation amount of the first head impact reducing member 151, which is a deformation amount occurring in the case 110 of the ultrasonic probe 200.

The deformation section S2 of the first head impact reducing member 151 may be configured to be positioned within the deformation section S1 of the first inner impact reducing member 241.

After a certain level of impact is applied to the first head part 1111 of the ultrasonic probe 200, the deformation section of the first inner impact reducing member 241 may decrease from S1 to S1'. Also, the deformation section of the first head impact reducing member 151 may decrease from S2 to S2'.

For example, in a case in which the first head impact reducing member 151 is deformed by 2 mm, when the first inner impact reducing member 241 is deformed by 1 mm, an impact corresponding to the remaining 1mm of deformation amount may be transmitted to the internal components. Therefore, the deformation amount occurring in the first inner impact reducing member 241 needs to be at least larger than or equal to the deformation amount of the first head impact reducing member 151.

Because an impact applied to the first head part 1111 may be very large, the first head impact reducing member 151 needs to be contracted to the limit it may be contracted, and the first inner impact reducing member 152 needs to absorb the remaining impact. Therefore, the deformation amount of the first inner impact reducing member 241 needs to be larger than the deformation amount of the first head impact reducing member 151.

Referring to FIGS. 11 and 12, the second inner impact reducing member 242 may be disposed between the second acoustic module 132 and the electronic circuit 120. The second inner impact reducing member 242 may include the second flexible circuit board 1421 provided to electrically connect the second acoustic module 132 and the electronic circuit 120, and the second elastic member 1422 disposed between the second acoustic module 132 and the electronic circuit 120.

The second elastic member 1422 may reduce an impact applied to the second head part 1112 from being transmitted to the electronic circuit 120 by being disposed between the second acoustic module 132 and the electronic circuit 120.

A deformation section S3 of the second inner impact reducing member 242 may be provided to be longer than or equal to a deformation section S4 of the second head impact reducing member 152. Also, a thickness of the second inner impact reducing member 242 may be provided to be thicker than a thickness of the second head impact reducing member 152.

The second head impact reducing member 152 may be positioned within the deformation section S3 of the second inner impact reducing member 242. That is, the second head impact reducing member 152 may be positioned within a section corresponding to the thickness of the second inner impact reducing member 242.

After a certain level of impact is applied to the second head part 1112 of the ultrasonic probe 200, the deformation section of the second inner impact reducing member 242 may decrease from S3 to S3'. Also, the deformation section of the second head impact reducing member 152 may decrease from S4 to S4'.

In this case as well, the deformation section S4 of the second head impact reducing member 152 may be provided to be positioned within the deformation section S3 of the second inner impact reducing member 242.

When configured as in the embodiment of the disclosure, the deformation amounts of the inner impact reducing members 241 and 242 may be larger than or equal to the deformation amounts of the head impact reducing members 151 and 152. Therefore, an impact transmitted to the internal components such as the electronic circuit 120 may be reduced more efficiently.

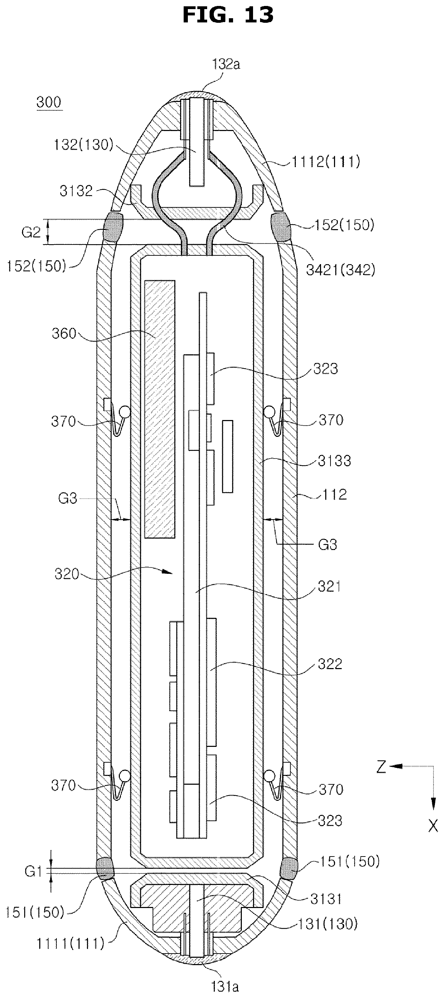

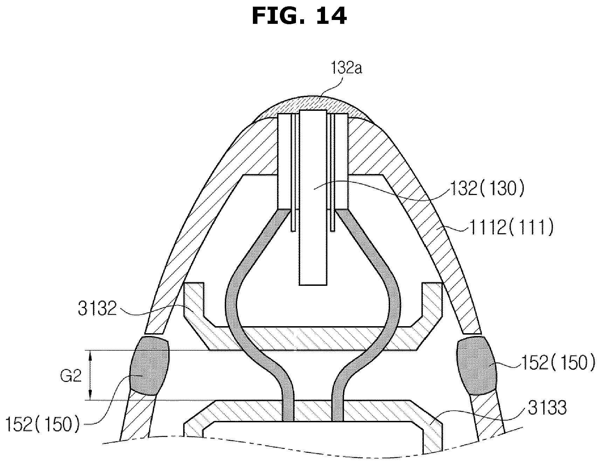

FIG. 13 is a cross-sectional view illustrating an internal structure of an ultrasonic probe according to an embodiment of the disclosure. FIG. 14 is an enlarged view of a portion of the ultrasonic probe of FIG. 13.

Referring to FIGS. 13 and 14, the ultrasonic probe 300 may include a handle part frame 3133 provided to cover the electronic circuit 120. The handle part frame 3133 may be disposed inside the handle part 112. The handle part frame 3133 may be provided to accommodate the electronic circuit 320. The handle part frame 3133 may be fixed to the handle part 112.

The electronic circuit 320 may be accommodated in the handle part frame 3133 disposed inside the handle part 112. That is, a main board 1321, a power board 322, a beamformer 323, and the like may be disposed inside the handle part 112. However, this is only an example, and the arrangement of the components inside the case 110 may be changed depending on design specifications.

The handle part frame 3133 may accommodate an energy storage device 360. That is, the energy storage device 360 may be disposed inside the handle part frame 3133. In this case, the energy storage device 360 is illustrated as a square battery, but is not limited thereto.

The ultrasonic probe 300 may include head part frames 3131 and 3132 provided to cover the acoustic module 130. The head part frames 3131 and 3132 may cover a portion of the acoustic module 130 that faces the handle part frame 3133. That is, the head part frames 3131 and 3132 may cover a portion of the acoustic module 130 opposite to portions where the head parts 1111 and 1112 are positioned.

The head part frames 3131 and 3132 may include the first head part frame 3131 provided to cover the first acoustic module 131, and the second head part frame 3132 provided to cover the second acoustic module 132.

The handle part frame 3133 and the head part frames 3131 and 3312 may include a material having high thermal conductivity to dissipate heat generated from the electronic circuit 320. The handle part frame 3133 and the head part frames 3131 and 3312 may also include a material having a certain rigidity sufficient to protect the electronic circuit 320. For example, the handle part frame 3133 and the head part frames 3131 and 3312 may include graphene or a graphite composite.

The first head part frame 3131 and the handle part frame 3133 may be disposed to be spaced apart from each other. Likewise, the second head part frame 3131 and the handle part frame 3133 may be disposed to be spaced apart from each other.

As the ultrasonic probe 300 is dropped or collides, the first head part 1111 may be impacted. The first head impact reducing member 151 may partially absorb the impact applied to the first head part 1111.

As the first head impact reducing member 151 is elastically deformed, the first head part 1111 may be moved in a direction in which the first head impact reducing member 151 is contracted. The first head part 1111 and the first head part frame 3131 may be integrally moved. That is, the first head part frame 3131 may also be moved in the direction in which the first head impact reducing member 151 is contracted.

In a case in which a gap G1 is not provided between the first head part frame 3131 and the handle part frame 3133, an impact may be transmitted to the handle part frame 3133.

In the embodiment of the disclosure, because the gap G1 is provided between the first head part frame 3131 and the handle part frame 3133, an impact applied to the first head part 1111 may be prevented from being transmitted to the handle part frame 3133. Therefore, the impact may be prevented from being transmitted to the electronic circuit 320 covered by the handle part frame 3133.

In another case, as the ultrasonic probe 300 is dropped or collides, the second head part 1112 may be impacted.

When the second head part 1112 is impacted, the second head impact reducing member 152 may partially absorb the impact. As the second head impact reducing member 152 is elastically deformed, the second head part 1112 may be moved in a direction in which the second head impact reducing member 152 is contracted. The second head part 1112 and the second head part frame 3132 may be integrally moved. That is, the second head part frame 3132 may also be moved in the direction in which the second head impact reducing member 152 is contracted.

The impact applied to the second head part 1112 may not be transmitted to the handle part frame 3133 due to a gap G2 provided between the second head part frame 3132 and the handle part frame 3133. Therefore, the impact may be prevented from being transmitted to the electronic circuit 320 covered by the handle part frame 3133.

In this case, an inner impact reducing member 342 including a flexible circuit board 3421 may be disposed between the second acoustic module 132 and the handle part frame 3133. The inner impact reducing member 342 may reduce an impact applied to the second head part 1112. The inner impact reducing member 342 is illustrated as including only the flexible circuit board 3421, but is not limited thereto and may further include an elastic member.

The flexible circuit board 3421 may electrically connect the second acoustic module 132 and the electronic circuit 320 positioned within the handle part frame 3133. The flexible circuit board 3421 may also reduce the impact applied to the second head part 1112.

In this embodiment, the inner impact reducing member 342 is illustrated as being disposed only between the second acoustic module 132 and the handle part frame 3133, but may also be disposed between the first acoustic module 131 and the handle part frame 3133.

In another case, as the ultrasonic probe 300 is dropped or collides, the handle part 112 may be impacted.

A gap G3 may be formed between the handle part 112 and the handle part frame 3133 so that the ultrasonic probe 300 may reduce an impact applied to the handle part 112. The ultrasonic probe 300 may also include a case elastic member 370 disposed between the handle part 112 and the handle part frame 3133. That is, the case elastic member 370 may be positioned in the gap G3 between the handle part 112 and the handle part frame 3133. The case elastic member 370 may be fixed to the inside of the handle part 112.

The case elastic member 370 may be elastically deformable. The case elastic member 370 may be provided in various forms such as a coil spring and a leaf spring. The impact applied to the handle part 112 may be reduced in the case elastic member 370. That is, the impact may be prevented from being transmitted to the electronic circuit 320 covered by the handle part frame 3133.

The gaps G1, G2, and G3 formed around the handle part frame 3133 may be heat dissipation paths for dissipating heat generated in the electronic circuit 320. Heat generated in the electronic circuit 320 may be dissipated through the handle part frame 3133 and moved through the gaps G1, G2, and G3 formed around the handle part frame 3133.

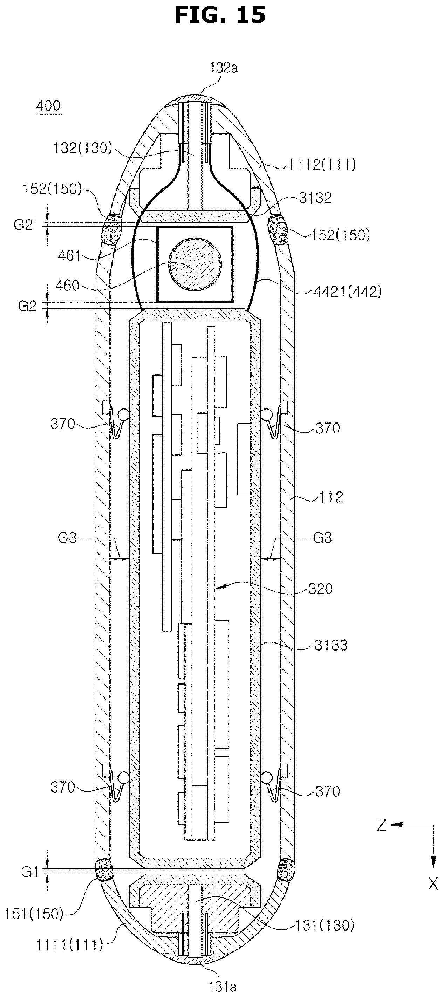

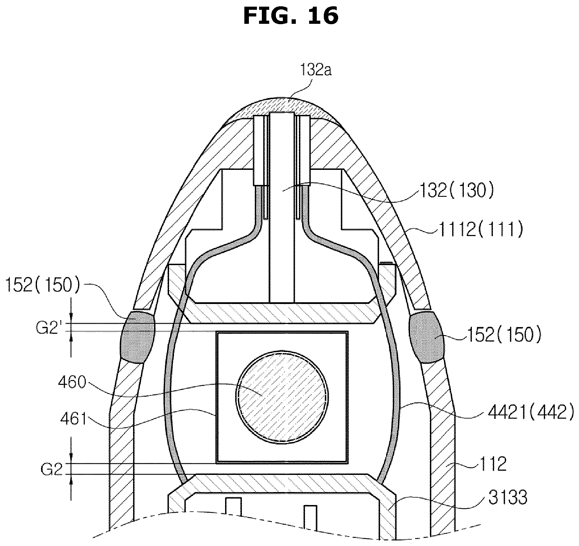

FIG. 15 is a cross-sectional view illustrating an internal structure of the ultrasonic probe according to an embodiment of the disclosure. FIG. 16 is an enlarged view of a portion of the ultrasonic probe of FIG. 15.

Referring to FIGS. 15 and 16, the ultrasonic probe 400 may include the second head part frame 3132 provided to cover the second acoustic module 132, and an energy storage device 460 disposed between the handle part frames 3133 provided to cover the electronic circuit 320.

The ultrasonic probe 400 may include an energy storage box 461 provided to cover the energy storage device 460. The energy storage box 461 may accommodate the energy storage device 460. The energy storage box 461 may include the same material as the handle part frame 3133 and the head part frames 3131 and 3132. For example, the energy storage box 461 may include graphene or a graphite composite.

The ultrasonic probe 400 may include an inner impact reducing member 442 disposed outside the energy storage box 461. The inner impact reducing member 442 may include a flexible circuit board 4421 disposed outside the energy storage box 461. That is, the flexible circuit board 4421 may be disposed outside the energy storage device 460.

Even when an impact is applied to the second head part 1112, the inner impact reducing member 442 may reduce the impact while being elastically deformed. Accordingly, the impact may be prevented from being transmitted to the energy storage device 460 disposed inside the inner impact reducing member 442. However, the inner impact reducing member 442 is not limited thereto, and may include an elastic member provided to surround the energy storage device 460, as described above.

The energy storage box 461 may be disposed to be spaced apart from the second head part frame 3132. The energy storage box 461 may also be disposed to be spaced apart from the handle part frame 3133.

The gap G2' formed between the energy storage box 461 and the second head part frame 3132 and the gap G2 formed between the energy storage box 461 and the handle part frame 3133 may be the same. However, both the gaps do not have to be the same size, and it is sufficient as long as the gaps are formed between the energy storage box 461, the handle part frame 3133, and the second head part frame 3132.

When an impact is applied to the second head part 1112 as the ultrasonic probe 400 is dropped or collides, the impact may be partially absorbed in the second head impact reducing member 152. At the same time, because the second head part 1112 is moved in the direction in which the second head impact reducing member 152 is contracted, the second head part frame 3132 may also be moved in the same direction.

Because the gap G2' is formed between the second head part frame 3132 and the energy storage box 461, an impact may be prevented from being transmitted to the energy storage box 461.

Because the gap G2' formed between the second head part frame 3132 and the energy storage box 461 may not be sufficient, an impact may be transmitted to the energy storage box 461. However, even in this case, the impact may be reduced due to the gap G2 formed between the energy storage box 461 and the handle part frame 3133.

Because the gap G2 is formed between the energy storage box 461 and the handle part frame 3133, the impact transmitted to the energy storage device 460 may also be reduced, and the impact may be prevented from being transmitted to the electronic circuit 320 covered by the handle part frame 3133.

The gap G2' formed between the energy storage box 461 and the second head part frame 3132 and the gap G2 formed between the energy storage box 461 and the handle part frame 3133 may be heat dissipation paths for dissipating heat.

Heat generated in the energy storage device 460 may be dissipated through the energy storage box 461, and flow through the gap G2 formed between the energy storage box 461 and the handle part frame 3133 and the gap G2' formed between the energy storage box 461 and the second head part frame 3132.

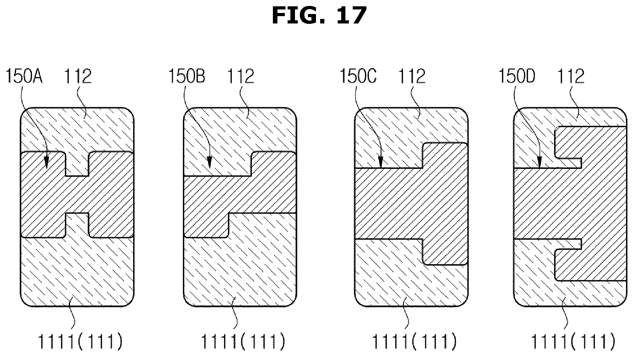

FIG. 17 is an enlarged view of part A of FIG. 4, which schematically illustrates various shapes capable of being provided as head impact reducing members.

Referring to FIG. 17, head impact reducing members 150A, 150B, 150C, and 150D may be provided in various shapes. In this embodiment, only a case in which the head impact reducing members 150A, 150B, 150C, and 150D are disposed between the first head part 1111 and the handle part 112 is illustrated, but likewise, a case in which the head impact reducing members 150A, 150B, 150C, and 150D are disposed between the second head part 1112 and the handle part 112 may also be provided. However, the disclosure is not limited thereto, and the head impact reducing members between the first head part 1111 and the handle part 112 and between the second head part 1112 and the handle part 112 may be provided in different shapes.

The head impact reducing member 150A may be provided between the first head part 1111 and the handle part 112. The head impact reducing member 150A may be provided such that inner and outer sides thereof are symmetrical.

The head impact reducing member 150B may have an inner side protruding toward the first head part 1111 and an outer side protruding toward the handle part 112. A portion of the first head part 1111 close to part A (see FIG. 4) may be impacted. In this case, an impact applied to the first head part 1111 may be efficiently absorbed only in a case in which the head impact reducing member 150B protrudes toward the handle part 112 as a cross-sectional shape thereof directs to the outside.

The head impact reducing member 150C may be provided such that an outer side thereof is thicker than an inner side thereof. Because the outer side of the head impact reducing member 150C is provided to be thicker, an impact applied to the head impact reducing member 150C may be absorbed more efficiently. In addition, when the first head part 1111 is coupled to the handle part 112, structural stability may also be improved.

The head impact reducing member 150D may be provided such that an outer side thereof is thicker than an inner side thereof, and may have a cross section in which a portion of the outer side protrudes toward the inner side. Because contact areas between the head impact reducing member 150D and the first head part 1111 and between the head impact reducing member 150D and the handle part 112 are expanded, the structural stability may be further improved. However, the cross-sectional shape of the head impact reducing member 150 is not limited thereto and may be provided in various shapes.

As is apparent from the above, an ultrasonic probe according to the disclosure can have improved durability.

The ultrasonic probe according to the disclosure can prevent damage to internal components from an external impact.

The ultrasonic probe according to the disclosure can include impact reducing members therein.

The foregoing has illustrated and described specific embodiments. However, it should be understood that the disclosure is not limited to the above-described embodiments, and various changes and modifications may be made without departing from the technical idea of the disclosure described in the following claims.

## Claims

1. An ultrasonic probe comprising:
a case comprising a handle part and a head part coupled to the handle part;
an acoustic module disposed inside the case to transmit an ultrasonic signal to an object and receive an echo signal reflected from the object;
an electronic circuit disposed inside the case to be electrically connected to the acoustic module; and
an inner impact reducing member disposed between the acoustic module and the electronic circuit to reduce an impact transmitted to the electronic circuit,
the probe being **characterized in that** the probe further comprises
a head impact reducing member disposed between the handle part and the head part,
wherein a deformation amount of the inner impact reducing member is provided to be larger than or equal to a deformation amount of the head impact reducing member, and
the head impact reducing member is positioned within a section corresponding to the deformation amount of the inner impact reducing member.

2. The ultrasonic probe according to claim 1, wherein
the inner impact reducing member further comprises a flexible circuit board disposed between the acoustic module and the electronic circuit to electrically connect the acoustic module and the electronic circuit.

3. The ultrasonic probe according to claim 2, further comprising
an energy storage device disposed between the acoustic module and the electronic circuit to supply electric power,
wherein the flexible circuit board is disposed outside the energy storage device to prevent an impact from being transmitted to the energy storage device.

4. The ultrasonic probe according to claim 1, wherein
the inner impact reducing member further comprises an elastic member disposed between the acoustic module and the electronic circuit.

5. The ultrasonic probe according to claim 4, further comprising
an energy storage device disposed between the acoustic module and the electronic circuit to supply electric power to the acoustic module or the electronic circuit,
wherein the elastic member is disposed outside the energy storage device to prevent an impact from being transmitted to the energy storage device.

6. The ultrasonic probe according to claim 5, wherein
a plurality of the elastic members is provided, and the plurality of elastic members is provided to be spaced apart along a longitudinal direction of the energy storage device.

7. The ultrasonic probe according to claim 1, wherein
the case further comprises a handle part frame disposed inside the handle part to cover the electronic circuit, and a head part frame disposed inside the head part to cover the acoustic module.

8. The ultrasonic probe according to claim 7, wherein
the handle part frame and the head part frame are disposed to be spaced apart from each other.

9. The ultrasonic probe according to claim 7, further comprising
a case elastic member disposed between the handle part frame and the handle part to reduce the impact transmitted to the electronic circuit.

10. The ultrasonic probe according to claim 9, further comprising
an energy storage device provided to supply electric power to the acoustic module or the electronic circuit,
wherein the energy storage device is disposed between the handle part frame and the head part frame to be spaced apart from the handle part frame and the head part frame, respectively.

11. The ultrasonic probe according to claim 1, wherein
the head part comprises a first head part coupled to one side of the handle part and a second head part coupled to the other side of the handle part, and
the acoustic module comprises a first acoustic module disposed in the first head part and a second acoustic module disposed in the second head part.

12. The ultrasonic probe according to claim 11, wherein
the inner impact reducing member comprises a first inner impact reducing member disposed between the first acoustic module and the electronic circuit, and a second inner impact reducing member disposed between the second acoustic module and the electronic circuit.

## Patentansprüche

1. Ultraschallsonde, die Folgendes aufweist:
ein Gehäuse mit einem Griffteil und einem mit dem Griffteil verbundenen Kopfteil;
ein akustisches Modul, das im Inneren des Gehäuses angeordnet ist, um ein Ultraschallsignal an ein Objekt zu senden und ein von dem Objekt reflektiertes Echosignal zu empfangen;
eine elektronische Schaltung, die innerhalb des Gehäuses angeordnet ist, um elektrisch mit dem akustischen Modul verbunden zu sein; und
ein inneres Aufprallminderungselement, das zwischen dem akustischen Modul und der elektronischen Schaltung angeordnet ist, um einen auf die elektronische Schaltung übertragenen Aufprall zu dämpfen,
wobei die Sonde **dadurch gekennzeichnet ist, dass** die Sonde des Weiteren Folgendes aufweist:
ein Kopfaufprallminderungselement, das zwischen dem Griffteil und dem Kopfteil angeordnet ist, wobei
ein Verformungsmaß des inneren Aufprallminderungselements größer oder gleich einem Verformungsmaß des Kopfaufprallminderungselements ist, und
das Kopfaufprallminderungselement innerhalb eines Abschnitts angeordnet ist, der dem Verformungsmaß des inneren Aufprallminderungselements entspricht.

2. Ultraschallsonde nach Anspruch 1, wobei
das innere Aufprallminderungselement des Weiteren eine flexible Leiterplatte aufweist, die zwischen dem akustischen Modul und der elektronischen Schaltung angeordnet ist, um das akustische Modul und die elektronische Schaltung elektrisch zu verbinden.

3. Ultraschallsonde nach Anspruch 2, die des Weiteren Folgendes aufweist:
eine Energiespeichervorrichtung, die zwischen dem akustischen Modul und der elektronischen Schaltung angeordnet ist, um elektrische Energie zu liefern,
wobei die flexible Leiterplatte außerhalb der Energiespeichervorrichtung angeordnet ist, um zu verhindern, dass ein Aufprall auf die Energiespeichervorrichtung übertragen wird.

4. Ultraschallsonde nach Anspruch 1, wobei
das innere Aufprallminderungselement des Weiteren ein elastisches Element aufweist, das zwischen dem akustischen Modul und der elektronischen Schaltung angeordnet ist.

5. Ultraschallsonde nach Anspruch 4, die des Weiteren Folgendes aufweist:
eine Energiespeichervorrichtung, die zwischen dem akustischen Modul und der elektronischen Schaltung angeordnet ist, um das akustische Modul oder die elektronische Schaltung mit elektrischer Energie zu versorgen,
wobei das elastische Element außerhalb der Energiespeichervorrichtung angeordnet ist, um zu verhindern, dass ein Aufprall auf die Energiespeichervorrichtung übertragen wird.

6. Ultraschallsonde nach Anspruch 5, wobei
eine Vielzahl an elastischen Elementen vorgesehen ist und die Vielzahl an elastischen Elementen so angeordnet ist, dass sie entlang einer Längsrichtung der Energiespeichervorrichtung beabstandet sind.

7. Ultraschallsonde nach Anspruch 1, wobei
das Gehäuse des Weiteren einen Griffteilrahmen, der innerhalb des Griffteils angeordnet ist, um die elektronische Schaltung abzudecken, und einen Kopfteilrahmen, der innerhalb des Kopfteils angeordnet ist, um das akustische Modul abzudecken, aufweist.

8. Ultraschallsonde nach Anspruch 7, wobei
der Griffteilrahmen und der Kopfteilrahmen so angeordnet sind, dass sie voneinander beabstandet sind.

9. Ultraschallsonde nach Anspruch 7, die des Weiteren Folgendes aufweist:
ein Gehäuse-Elastikelement, das zwischen dem Griffteilrahmen und dem Griffteil angeordnet ist, um den auf die elektronische Schaltung übertragenen Aufprall zu reduzieren.

10. Ultraschallsonde nach Anspruch 9, die des Weiteren Folgendes aufweist:
eine Energiespeichervorrichtung, die dafür vorgesehen ist, das akustische Modul oder die elektronische Schaltung mit elektrischer Energie zu versorgen,
wobei die Energiespeichervorrichtung zwischen dem Griffteilrahmen und dem Kopfteilrahmen angeordnet ist, um jeweils von dem Griffteilrahmen und von dem Kopfteilrahmen beabstandet zu sein.

11. Ultraschallsonde nach Anspruch 1, wobei
der Kopfteil einen ersten Kopfteil, der mit einer Seite des Griffteils verbunden ist, und einen zweiten Kopfteil, der mit der anderen Seite des Griffteils verbunden ist, aufweist, und
das akustische Modul ein erstes akustisches Modul, das im ersten Kopfteil angeordnet ist, und ein zweites akustisches Modul, das im zweiten Kopfteil angeordnet ist, aufweist.

12. Ultraschallsonde nach Anspruch 11, wobei
das innere Aufprallminderungselement ein erstes inneres Aufprallminderungselement, das zwischen dem ersten akustischen Modul und der elektronischen Schaltung angeordnet ist, und ein zweites inneres Aufprallminderungselement, das zwischen dem zweiten akustischen Modul und der elektronischen Schaltung angeordnet ist, aufweist.

## Revendications

1. Sonde à ultrasons, comprenant :
un boîtier comprenant une partie de poignée et une partie de tête couplée à la partie de poignée ;
un module acoustique disposé à l'intérieur du boîtier pour transmettre un signal à ultrasons vers un objet et recevoir un signal d'écho réfléchi depuis l'objet ;
un circuit électronique disposé à l'intérieur du boîtier pour être connecté électriquement au module acoustique ; et
un élément de réduction d'impact interne disposé entre le module acoustique et le circuit électronique pour réduire un impact transmis au circuit électronique,
la sonde étant **caractérisée en ce que** la sonde comprend en outre :
un élément de réduction d'impact de tête disposé entre la partie de poignée et la partie de tête, dans laquelle
une quantité de déformation de l'élément de réduction d'impact interne est prévue pour être supérieure ou égale à une quantité de déformation de l'élément de réduction d'impact de tête, et
l'élément de réduction d'impact de tête est positionné à l'intérieur d'une section correspondant à la quantité de déformation de l'élément de réduction d'impact interne.

2. Sonde à ultrasons selon la revendication 1, dans laquelle
l'élément de réduction d'impact interne comprend en outre une carte de circuit imprimé flexible disposée entre le module acoustique et le circuit électronique pour connecter électriquement le module acoustique et le circuit électronique.

3. Sonde à ultrasons selon la revendication 2, comprenant en outre :
un dispositif de stockage d'énergie disposé entre le module acoustique et le circuit électronique pour fournir une alimentation électrique,
dans laquelle la carte de circuit imprimé flexible est disposée à l'extérieur du dispositif de stockage d'énergie pour empêcher un impact d'être transmis au dispositif de stockage d'énergie.

4. Sonde à ultrasons selon la revendication 1, dans laquelle
l'élément de réduction d'impact interne comprend en outre un élément élastique disposé entre le module acoustique et le circuit électronique.

5. Sonde à ultrasons selon la revendication 4, comprenant en outre :
un dispositif de stockage d'énergie disposé entre le module acoustique et le circuit électronique pour alimenter électriquement le module acoustique ou le circuit électronique,
dans laquelle l'élément élastique est disposé à l'extérieur du dispositif de stockage d'énergie pour empêcher un impact d'être transmis au dispositif de stockage d'énergie.

6. Sonde à ultrasons selon la revendication 5, dans laquelle
une pluralité des éléments élastiques sont prévus, et la pluralité d'éléments élastiques sont prévus pour être espacée le long d'une direction longitudinale du dispositif de stockage d'énergie.

7. Sonde à ultrasons selon la revendication 1, dans laquelle
le boîtier comprend en outre un cadre de partie de poignée disposé à l'intérieur de la partie de poignée pour recouvrir le circuit électronique, et un cadre de partie de tête disposé à l'intérieur de la partie de tête pour recouvrir le module acoustique.

8. Sonde à ultrasons selon la revendication 7, dans laquelle
le cadre de partie de poignée et le cadre de partie de tête sont disposés pour être espacés l'un de l'autre.

9. Sonde à ultrasons selon la revendication 7, comprenant en outre :
un élément élastique de boîtier disposé entre le cadre de partie de poignée et la partie de poignée pour réduire l'impact transmis au circuit électronique.

10. Sonde à ultrasons selon la revendication 9, comprenant en outre :
un dispositif de stockage d'énergie prévu pour alimenter électriquement le module acoustique ou le circuit électronique,
dans laquelle le dispositif de stockage d'énergie est disposé entre le cadre de partie de poignée et le cadre de partie de tête pour être espacé du cadre de partie de poignée et du cadre de partie de tête, respectivement.

11. Sonde à ultrasons selon la revendication 1, dans laquelle
la partie de tête comprend une première partie de tête couplée à un côté de la partie de poignée et une seconde partie de tête couplée à l'autre côté de la partie de poignée, et
le module acoustique comprend un premier module acoustique disposé dans la première partie de tête et un second module acoustique disposé dans la seconde partie de tête.

12. Sonde à ultrasons selon la revendication 11, dans laquelle
l'élément de réduction d'impact interne comprend un premier élément de réduction d'impact interne disposé entre le premier module acoustique et le circuit électronique, et un second élément de réduction d'impact interne disposé entre le second module acoustique et le circuit électronique.
